# EUROPÄISCHE PATENTANMELDUNG

(11) **EP 4 088 784 A1**
(43) Veröffentlichungstag der Anmeldung: **16.11.2022**
(21) Anmeldenummer: 22167859.2
(22) Anmeldetag: 12.04.2022
(51) Int. Cl.: A61Q 17/00, A61K 8/73, A61K 8/43, A61K 8/84, A61K 8/362, A61K 8/365, A61K 8/368, A61K 31/60, A61P 17/00, A61P 31/02

(54) **ZUSAMMENSETZUNG ZUR DESINFEKTION UND FÜR KOSMETISCHE ANWENDUNGEN**

(30) Priorität: 19.04.2021 DE 102021109834
(71) Anmelder: Fraunhofer-Gesellschaft zur Förderung der angewandten Forschung e.V., 80686 München (DE)
(72) Erfinder: Jelen, Erich, 46047 Oberhausen (DE)
(74) Vertreter: Freiherr von Foullon, Alexander

(57) **Zusammenfassung**

Die vorliegende Erfindung betrifft eine wässrige Zusammensetzung zur Desinfektion und für kosmetische Anwendungen, ihre Verwendung und ein Verfahren zu ihrer Herstellung.

## Beschreibung

Die vorliegende Erfindung betrifft das technische Gebiet der Kosmetika sowie der Desinfektionsmittel.

Insbesondere betrifft die vorliegende Erfindung eine wässrige Zusammensetzung, welche sich zur Verwendung in oder als Kosmetika und in oder als Desinfektionsmittel eignet.

Weiterhin betrifft die vorliegende Erfindung die Verwendung der Zusammensetzung in oder als Kosmetika sowie in oder als Desinfektionsmittel.

Weiterhin betrifft die vorliegende Erfindung Kosmetika und Desinfektionsmittel, welche die wässrige Zusammensetzung enthalten.

Schließlich betrifft die vorliegende Erfindung ein Verfahren zur Herstellung einer wässrigen Zusammensetzung, welche sich zur Verwendung in oder als Kosmetika sowie zur Verwendung in oder als Desinfektionsmittel eignet.

Unter Desinfektion versteht man im Allgemeinen die Entseuchung, d.h. die Abtötung pathogener Erreger an Organismen oder Gegenständen durch chemische Mittel oder physikalische Verfahren. Als physikalische Verfahren sind dabei insbesondere ionisierende Strahlung, Ultraschall oder auch Ultraviolettstrahlung zu nennen, welche direkt auf pathogene Erreger einwirken und diese abtöten bzw. inaktivieren.

Chemische Mittel zur Desinfektion werden auch als Desinfektionsmittel bezeichnet und im Allgemeinen zur Desinfektion von Oberflächen der Haut, der Kleidung, von Geräten oder Räumen eingesetzt, aber auch zur Desinfektion von Trinkwasser, Nahrungsmitteln oder Saatgut.

Desinfektionsmittel werden üblicherweise definiert als Stoffe oder Stoffgemische, die bei einer Anwendung auf Gegenständen oder Oberflächen diese in einen Zustand versetzen, dass sie keine Infektionen mehr verursachen können. Die Wirkung eines Desinfektionsmittels ist vorzugsweise allgemein mikrobizid, d.h. bakterizid, fungizid, viruzid und sporizid, weshalb die Anwendung von Desinfektionsmitteln auch als Sterilisation mit chemischen Mitteln bezeichnet wird. Hochwirksame Desinfektionsmittel sind somit pantoxisch, d.h. sie entfalten ihre Wirkung gegen alle lebenden Zellen.

Eine besondere Stellung unter den Desinfektionsmitteln nehmen lokal anzuwendende Desinfektionsmittel zur Wunddesinfektion ein, welche auch als Antiseptika bezeichnet werden.

Das Anforderungsprofil an Desinfektionsmitteln ist breit gefächert: So wird insbesondere ein breites Wirkungsspektrum gefordert, bei gleichzeitig geringer Toxizität, hoher Hautverträglichkeit, guter Materialverträglichkeit sowie geringen Einwirkungszeiten.

Ein breites Wirkungsspektrum bei gleichzeitig geringer Toxizität und guter Hautverträglichkeit ist für Anwendungen in Kosmetika, Antiseptika oder Handdesinfektionsmittel zwingende Voraussetzungen.

Es ist eine Vielzahl von Desinfektionsmitteln für unterschiedlichste Anwendungen bekannt. In der Praxis werden oftmals Aldehyde, wie beispielsweise Formaldehyd oder Glutaraldehyd, aber auch Phenolderivate eingesetzt. Gängige Desinfektionsmittel sind weiterhin hochkonzentrierte Alkohole, insbesondere in Konzentrationen von 40 bis 80 Vol.-%. In Kosmetika werden oftmals auch quartäre Ammoniumverbindungen und Kationentenside eingesetzt, welche eine gute Haut- und Materialverträglichkeit aufweisen sowie geruchsneutral sind, allerdings in ihrem Wirkspektrum stark begrenzt sind. Typische Vertreter für Ammoniumverbindungen sind insbesondere Benzalkoniumchlorid oder Hexadecylpyridiniumchlorid.

Auch Halogene, wie Chlor und Iod, oder auch Sauerstoff, insbesondere in Form von Wasserstoffperoxid oder Peressigsäure, sind gängige Desinfektionsmittel, welche zur Flächendesinfektion verwendet werden.

Problematisch ist bei der Desinfektion von Oberflächen, insbesondere beispielsweise im öffentlichen Raum oder auf der Haut oder bei der Wunddesinfektion, dass nach der erfolgten Desinfektion schnell eine Rekontamination mit Erregern aus der Umgebung eintritt. Die Desinfektionsmittel sind zwar bei Auftrag auf die Oberfläche oftmals hochwirksam, jedoch wird das Desinfektionsmittel rasch von der Oberfläche entfernt, beispielsweise durch Verdunstung oder Abwischen, so dass mit einer Rekontaminierung zu rechnen ist.

Aus diesem Grund wurden filmbildende Desinfektionsmittel entwickelt, welche in der Regel in Form von Lösungen oder Dispersionen aufgebracht werden und nach Verflüchtigung des Löse- oder Dispersionsmittels auf der Oberfläche verbleiben und dort für eine länger andauernde mikrobizide Wirkung sorgen. Speziell für filmbildende Desinfektionsmittel gilt besonders, dass diese eine nur geringe Toxizität aufweisen dürfen und darüber hinaus haut- und materialverträglich sein müssen und insbesondere die Eigenschaften der Oberflächen, auf welche Sie aufgebracht werden, nicht nachteilig beeinflussen.

In zunehmendem Maße werden zur Desinfektion auch Biopolymere, insbesondere Chitosan, eingesetzt, welche filmbildende Eigenschaften aufweist. Chitosan auch Polyglusam oder Poly-D-Glucosamin genannt, ist ein natürlich vorkommendes Biopolymer, welches sich von Chitin ableitet. Chitosan ist ein Polyaminosaccharid, welches technisch durch Deacetylierung aus Chitin gewonnen wird. Die Deacetylierung kann dabei entweder durch Natronlauge oder auch enzymatisch erfolgen. Chitin ist aus etwa 2000 linear miteinander verbunden 2-Glucosamineinheiten, d.h. 2-Amino-2-desoxy-β-D-glucopyranose-Einheiten, aufgebaut und Chitosan unterscheidet sich von Chitin durch einen variablen Deacetylierungsgrad. Chitosan wird insbesondere technisch zur Bindung von Schwebstoffen als Filtermedium eingesetzt, aber auch zur Herstellung von Fasern, Schaumstoffen oder Membranen. Chitosan weist darüber hinaus koagulierende, blutstillende Eigenschaften auf, wirkt abrasiv, aber auch oberflächenglättend und in Maßen antibakteriell und antiviral. Aus diesen Gründen wird Chitosan im medizinischen Bereich für Wundauflagen, aber auch in Zahnpasta eingesetzt. Ein Nachteil von Chitosan ist, dass es zwar antiviral wirkt, jedoch nur bakteriostatisch oder fungistatisch, d.h. Bakterien und Pilze werden in ihrem Wachstum zwar gehemmt, aber nicht inaktiviert bzw. abgetötet. Aufgrund des nur eingeschränkten Wirkspektrums wird Chitosan trotz seiner geringen Toxizität und hoher Verträglichkeit insgesamt nur selten als Desinfektionsmittel eingesetzt. Auch andere filmbildende Biopolymere werden nur selten als Desinfektionsmittel eingesetzt.

Filmbildende Eigenschaften sind jedoch nicht nur im Bereich der Desinfektionsmittel, sondern auch im Bereich der Kosmetika erwünscht, insbesondere in Haut- oder Haar-Pflegeprodukten, weshalb Chitosan und auch andere Biopolymere, wie beispielsweise Hyaluronsäure, in Haut- und Haarpflegeprodukten verwendet werden. Chitosan ist nur schlecht in Wasser löslich, löst sich jedoch gut in Säuren, sowohl in anorganischen als auch in organischen Säuren, und wird daher oftmals in Zusammensetzungen in Form von Chitosansalicylaten oder -zitronaten eingesetzt.

Salicylsäure bzw. 2-Hydroxybenzencarbonsäure oder 2-Hydroxybenzoesäure besitzt wie Chitosan eine gewisse antimikrobielle Wirkung, ist jedoch relativ toxisch bei oraler Aufnahme und führt darüber hinaus an Haut, Schleimhäuten und Augen zu Reizungen und Gewebeschädigungen. Weiterhin verursacht Salicylsäure in höheren Dosen auch Schädigungen des zentralen Nervensystems.

Aus diesem Grund ist der Einsatz von Salicylsäure als allgemeiner Konservierungsstoff verboten. In kosmetischen Mitteln ist ihr Einsatz mit einer Konzentration von maximal 3 % in Antischuppenmitteln und mit einer Konzentration bis zu 2 % in sonstigen Mitteln zugelassen sowie als Konservierungsstoff in Kosmetika mit maximal 0,5 %.

Insbesondere im Bereich der Kosmetik, der Dermatologie und dem Pflanzenschutz sind Kombinationen der Einzelsubstanzen Salicylsäure und Chitosan seit langem bekannt, da - wie zuvor ausgeführt - jede Substanz für sich genommen antimikrobielle oder generell konservierende Eigenschaften besitzt, und dies auch für ihre Kombination gilt. Da sowohl Chitosan als auch Salicylsäure natürlich vorkommende Stoffe und auch im großen Maße verfügbar sind, hat es im Stand der Technik nicht an Versuchen gefehlt, sie für kosmetische Anwendungen oder Anwendungen in Desinfektionsmitteln nutzbar zu machen.

So beschreibt die CN 104622710 A alkoholische, insbesondere ethanolische Zusammensetzungen mit Chitosan und Salicylsäure, welche ein spezielles Gewichtsverhältnis von Salicylsäure zu Chitosan aufweisen, das in einem Bereich oberhalb von 1 : 1 liegt. Die beschriebenen Zusammensetzungen enthalten stets Ethanol oder ein Polyol, wie beispielsweise Glycerin, als Lösungsvermittler.

Weiterhin betrifft die WO 02/49604 A1 eine Konservierungsmittelzusammensetzung für Feuchttücher, welche eine alkoholische Zusammensetzung aus Chitosan und Salicylsäure enthält.

Weiterhin beschreibt die US 2008/0045491 A1 ein Desinfektionsmittel, welches Chitosan und Salicylsäure enthält.

Die DE 10 2008 046 207 A1 betrifft ein natürliches Haarbehandlungsmittel, welches Chitosan und/oder Salicylsäure aufweisen kann.

Als nachteilig bei der Verwendung von Chitosan oder Salicylsäure ist stets festzustellen, dass sowohl Salicylsäure als auch Chitosan nahezu wasserunlöslich sind. Für eine antimikrobielle Wirkung oder auch für die Filmbildung ist jedoch eine Wasserlöslichkeit oder zumindest Wasserdispergierbarkeit unabdingbar, um in einem für Erreger anfälligen Medium aktiv zu sein. Nachteilig ist weiterhin, dass die salzartigen Verbindungen von Chitosan und Salicylsäure, welche sich beim Mischen der Verbindungen ergeben, aus wässrigen Lösungen rasch auskristallisieren und nicht langzeitstabil sind.

Es fehlt somit sowohl im kosmetischen Bereich als auch im Bereich der Desinfektionsmittel weiterhin an Zusammensetzungen, welche in Lösungen oder Dispersionen langzeitstabil und filmbildend sind, eine geringe Toxizität sowie keine anderen unerwünschten Nebenwirkungen aufweisen und oberflächen- und haut- bzw. haarverträglich sind.

Für Desinfektionsmittel oder für Mittel für die kombinierte Anwendungen in Desinfektion oder Kosmetik ist darüber hinaus auch ein möglichst breites Wirkungsspektrum an mikrobiziden Eigenschaften erwünscht.

Wünschenswert wäre weiterhin eine kosmetische Zusammensetzung bzw. eine Zusammensetzung zur Anwendung in Kosmetika, welche hervorragende filmbildende Eigenschaften aufweist und in der Lage ist, Haut und Haare zu schützen.

Gleichfalls wäre es wünschenswert, ein Desinfektionsmittel zur Hand zu haben, welches stabile Filme bildet und bei großem Wirkspektrum eine geringe Toxizität aufweist und hervorragende Langzeitwirkungen besitzt, d.h. auch zur dauerhaften Desinfektion von Oberflächen geeignet ist.

Es ist somit eine Aufgabe der vorliegenden Erfindung, die zuvor geschilderten mit dem Stand der Technik verknüpften Probleme und Nachteile zu überwinden oder diese zumindest jedoch abzuschwächen.

Insbesondere ist eine Aufgabe der vorliegenden Erfindung darin zu sehen, eine Zusammensetzung bereitzustellen, welche vorzugsweise ohne organische Lösemittel auskommt, hervorragende Filmbildungseigenschaften aufweist und sowohl für kosmetische Anwendungen als auch zur Desinfektion eingesetzt werden kann.

Eine weitere Aufgabe der vorliegenden Erfindung ist darin zu sehen, eine Zusammensetzung für kosmetische Anwendungen bereitzustellen, welche eine geringe Toxizität und hervorragende haut- und haarpflegende Eigenschaften aufweist.

Weiterhin ist eine Aufgabe der vorliegenden Erfindung darin zu sehen, ein Desinfektionsmittel bereitzustellen, welches hervorragende filmbildende Eigenschaften, eine geringe Toxizität bei breitem Wirkungsspektrum und eine hervorragende Langzeitwirkung aufweist.

Gegenstand der vorliegenden Erfindung gemäß einem ersten Aspekt der vorliegenden Erfindung ist eine wässrige Zusammensetzung gemäß Anspruch 1; weitere vorteilhafte Ausgestaltungen dieses Erfindungsaspektes sind Gegenstand der diesbezüglichen Unteransprüche.

Weiterer Gegenstand der vorliegenden Erfindung gemäß einem zweiten Aspekt der vorliegenden Erfindung ist die Verwendung einer wässrigen Zusammensetzung in oder als Kosmetika nach Anspruch 10.

Weiterer Gegenstand der vorliegenden Erfindung gemäß einem dritten Aspekt der vorliegenden Erfindung ist die Verwendung einer wässrigen Zusammensetzung in oder als Desinfektionsmittel nach Anspruch 11.

Wiederum weiterer Gegenstand der vorliegenden Erfindung gemäß einem vierten Aspekt der vorliegenden Erfindung ist eine kosmetische Zubereitung nach Anspruch 12.

Wiederum weiterer Gegenstand der vorliegenden Erfindung gemäß einem fünften Aspekt der vorliegenden Erfindung ist Desinfektionsmittel nach Anspruch 13.

Schließlich ist weiterer Gegenstand der vorliegenden Erfindung gemäß einem sechsten Aspekt der vorliegenden Erfindung ein Verfahren zur Herstellung einer wässrigen Zusammensetzung nach Anspruch 14, weitere vorteilhafte Ausgestaltungen dieses Erfindungsaspekts sind Gegenstand der diesbezüglichen Unteransprüche.

Es versteht sich von selbst, dass im Folgenden genannte, besondere Ausgestaltungen, insbesondere besondere Ausführungsformen oder dergleichen, welche nur in Bezug mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der Inhaltsstoffe, Zusatz- bzw. Hilfsstoffe oder dergleichen stets 100 % bzw. 100 Gew.-% resultieren. Dies versteht sich für den Fachmann aber von selbst.

Zudem gilt, dass alle im Folgenden genannten Parameterangaben oder dergleichen grundsätzlich mit genormten oder explizit angegebenen Bestimmungsverfahren oder mit für den Fachmann an sich geläufigen Bestimmungsmethoden bestimmt bzw. ermittelt werden können.

Dies vorausgeschickt wird nachfolgend der Gegenstand der vorliegenden Erfindung näher erläutert.

Gegenstand der vorliegenden Erfindung - gemäß einem **ersten** Aspekt der vorliegenden Erfindung - ist somit eine wässrige Zusammensetzung, insbesondere für Hygiene- und/oder Kosmetikanwendungen, wobei die Zusammensetzung einen Komplex aus
a) mindestens einem Polysaccharid, welches Monomereinheiten aus gegebenfalls substituierten Aminozuckern, insbesondere Glucosaminen und oder Galactosaminen, aufweist,
b) mindestens einer Carbonsäure und
c) mindestens einem Polyamin und/oder einem Guanid, insbesondere Biguanid aufweist.

Denn, wie die Anmelderin überraschenderweise herausgefunden hat, sind mit der erfindungsgemäßen Zusammensetzung, insbesondere dem erfindungsgemäß eingesetzten Komplex, Zusammensetzungen zugänglich, welche hervorragende Filmbildungseigenschaften aufweisen und sich sowohl für kosmetische als auch für desinfizierende Anwendungen eignen. Der erfindungsgemäß eingesetzte Komplex weist dabei nicht nur eine hervorragende Löslichkeit bzw. Solubilisierbarkeit in Wasser auf, sondern ist darüber hinaus als wässrige Zusammensetzung langzeitstabil lagerbar. Insbesondere sind die erfindungsgemäßen wässrigen Zusammensetzungen, welche den Komplex enthalten, über Monate stabil lagerbar, im Gegensatz zu üblichen Chitosan-Salicylaten.

Durch die herausragenden filmbildenden Eigenschaften des erfindungsgemäßen Komplexes ist es insbesondere möglich, kosmetische Zusammensetzungen herzustellen, welche sowohl haut- und haarschützend als auch wundheilfördernd sind.

Gleichermaßen ist es möglich, mit dem erfindungsgemäßen Komplex nicht nur ein spontan, d.h. sofort, wirkendes Desinfektionsmittel bereitzustellen, sondern - aufgrund der ausgeprägten Filmbildungseigenschaften - ein Desinfektionsmittel, welches eine hervorragende Langzeitwirkung aufweist und bei Aufbringung auf Oberflächen langanhaltend für Keimfreiheit oder zumindest eine Keimreduzierung sorgt.

Die erfindungsgemäße Zusammensetzung mit dem spezifischen Komplex ist dabei insbesondere aus nicht-toxischen Substanzen aufgebaut bzw. es können aufgrund der hohen Wirksamkeit und der synergistischen Effekte prinzipiell toxische Substanzen in Mengen angewendet werden, in welchen sie nicht toxisch wirken, so dass die erfindungsgemäße Zusammensetzung auch bedenkenlos zur Desinfektion von Lebensmitteln oder in Betrieben zur Verarbeitung von Lebensmitteln, wie beispielsweise Bäckereien, Metzgereien oder Fleischereien, Anwendung finden kann.

Wenn die erfindungsgemäße Zusammensetzung in oder als Desinfektionsmittel angewendet werden soll, kommen - wie nachfolgend noch ausführlich beschrieben - vorzugsweise Polysaccharide zum Einsatz, welche intrinsisch schon gewisse mikrobizide Eigenschaften aufweisen, wie beispielsweise Chitosan.

Durch die Kombination des Polysaccharids, d.h. eines Biopolymers, und der Carbonsäure mit einem Polyamin bzw. Guanid oder Biguanid wird zum einen die Stabilität des Komplexes erhöht, zum anderen werden auch die filmbildenden Eigenschaften sowie mikrobizide Eigenschaften der Zusammensetzung, insbesondere bei Auswahl geeigneter Polyamine oder Biguanide, deutlich verbessert.

Ohne sich auf diese Theorie festlegen zu wollen, scheint die Multifunktionalität des Polyamins bzw. Guanids oder Biguanids notwendig zu sein, um diese Verbesserung zu erzielen, da beispielsweise die Verwendung einfacher Amine nicht die gewünschte Wirkung zeigt. Durch den gezielten Einsatz von Polyaminen oder Guaniden bzw. Biguaniden, welche beispielsweise desinfizierende Eigenschaften aufweisen, wie zum Beispiel Polyhexanid (Polyhexamethylenbiguanid) kann die desinfizierende Wirkung des Komplexes deutlich verstärkt werden, wobei das desinfizierende Polyamid oder Guanid bzw. Biguanid mit mikrobioziden Eigenschaften in deutlich geringeren Mengen als üblicherweise vorgesehen eingesetzt werden kann und trotzdem hervorragende Desinfektionswirkungen erzielt werden.

Aufgrund der herausragenden Filmbildungseigenschaften der erfindungsgemäßen wässrigen Zusammensetzung kann diese beispielsweise als Foam-Booster und Konditionierer eingesetzt, welche sowohl die Haarstruktur als auch die Reinigungsleistung eines Shampoos deutlich verbessern, weshalb bei Anwendung der erfindungsgemäßen Zusammensetzung in Shampoos üblicherweise auf einen zweiten Waschgang verzichtet werden kann. Die erfindungsgemäße wässrige Zusammensetzung mit dem spezifischen Komplex kann hierbei insbesondere als Substitut für Silikone oder Mikroplastik eingesetzt werden, was zum einen deutliche anwendungstechnische Vorteile bringt und zum anderen auch mit einer stark verbesserten Umweltbilanz einhergeht.

Die erfindungsgemäße wässrige Zusammensetzung eignet sich insbesondere auch für einen substantiellen Haaraufbau, da der Haardurchmesser durch Applikation der Zusammensetzung deutlich vergrößert werden kann. Gleichzeitig wird eine verbesserte Kämmbarkeit erzielt, insbesondere auch bei thermischem Stress. Darüber hinaus werden auch ein verbesserter Haarglanz sowie sehr gute Antifrizz-Eigenschaften beobachten; dies gilt überraschenderweise auch bei thermischem Stress, wie er beispielsweise während des Föhnens vorkommt, bei welchem das Haar oftmals Temperaturen von über 50 °C ausgesetzt ist.

Darüber hinaus wird auch eine innerstrukturelle Restrukturierung des Haars bei Anwendung der erfindungsgemäßen wässrigen Zusammensetzung in Shampoos beobachtet, welche zu einer Erhöhung des Schmelzbereichs sowie der Schmelzenthalpie des Haares führt.

Weiterhin wird ein Lotuseffekt auf dem Haar oder auf anderen Oberflächen, auf welche die Zusammensetzung aufgebracht wird, beobachtet, welcher einer Verschmutzung entgegenwirkt. Gleichermaßen wird auch die Wasserbindung im Haar signifikant verbessert, d.h. die Moisture-Balance wird verbessert.

Darüber hinaus ist als vorteilhaft anzusehen, dass bei Anwendung der erfindungsgemäßen Zusammensetzung in Shampoos oder Waschlotionen mit dem normalen Waschen auch ein Desinfizieren des Haares oder der Haut vorgesehen sein kann, und dies unter ansonsten sehr milden, verträglichen Bedingungen, da die erfindungsgemäße wässrige Zusammensetzung hervorragend haut- und haarverträglich ist.

Auch die Haut wird durch die verbesserten Filmbildungseigenschaften bei Anwendung der erfindungsgemäßen wässrigen Zusammensetzung effektiv vor äußeren Einflüssen geschützt und auch wundheilfördernde Auswirkungen lassen sich beobachten. Aufgrund der guten Verträglichkeit eignet sich die erfindungsgemäße Zusammensetzung sowohl in Kosmetika als auch in Desinfektionsmitteln zur täglichen Anwendung auf Haut und Haar.

Unter einem "Komplex" sind im Rahmen der vorliegenden Erfindung nicht nur anorganische bzw. metallorganische Koordinationsverbindungen oder spezifische molekulare organische Verbindungen zu verstehen, sondern auch Gemische aus verschiedenen Substanzen zwischen denen chemische und/oder physikalische Wechselwirkungen vorliegen, wie beispielsweise Ionenbindungen, kovalente Bindungen, oder Dipol-Dipol-Wechselwirkungen, oder van-der-Waals-Wechselwirkungen, so dass die Gemische nach außen als Ganzes wirken. Dies bedeutet, dass die Eigenschaften der Einzelsubstanzen nach außen oftmals nicht mehr zu erkennen sind, sondern die Eigenschaften der Mischung dominieren. Beispielsweise können die Löslichkeiten in Wasser oder anderen Lösemitteln durch Komplexbildung positiv oder negativ beeinflusst werden. Auch das Desinfektionsvermögen bzw. die Filmbildungseigenschaften können deutlich verändert sein. Entscheidend ist, dass zwischen den Substanzen eine augenscheinliche Interaktion vorliegt, welche die Eigenschaften der Einzelsubstanzen ändert.

Im Rahmen der vorliegenden Erfindung ist es üblicherweise vorgesehen, dass der Komplex in der Zusammensetzung gelöst oder dispergiert vorliegt.

Unter einer Lösung ist im Rahmen der vorliegenden Erfindung insbesondere ein einphasiges System zu verstehen, in welchem Moleküle, deren Bestandteile oder auch Komplexe vereinzelt und homogen verteilt vorliegen.

Unter einer Dispersion ist im Rahmen der vorliegenden Erfindung ein mindestens zweiphasiges System zu verstehen, in welchem eine erste Phase, die dispergierte Phase, in feiner Verteilung in einer zweiten Phase, der kontinuierlichen Phase, dem sogenannten Dispersionsmedium, vorliegt. Die dispergierte Phase bildet dabei oftmals Agglomerate aus einzelnen Molekülen bzw. Komplexen in der kontinuierlichen Phase. Insbesondere im Bereich der makromolekularen Chemie sind die Übergänge von Lösungen zu Dispersionen fließend, d.h. sobald Makromoleküle beteiligt sind, kann oftmals nicht mehr scharf zwischen Lösungen und Dispersionen unterschieden werden.

Im Rahmen der vorliegenden Erfindung ist es üblicherweise vorgesehen, dass die Zusammensetzung, insbesondere die Lösung oder Dispersion, in Form eines Gels, insbesondere eines stabilen Gels, vorzugsweise eines Hydrogels, vorliegt.

Unter dem Begriff "Gel" werden im Rahmen der vorliegenden Erfindung vorzugsweise leicht deformierbare an Flüssigkeiten und Gasen reiche disperse Systeme aus mindestens zwei Komponenten, die zumindest aus einem festen, kolloidbildenden Stoff mit langen oder stark verzweigten Teilchen, insbesondere auch Polysacchariden, und einer Flüssigkeit, wie beispielsweise Wasser, als Dispersionsmittel bestehen. Dabei ist die feste Substanz kohärent, d.h. sie bildet in Dispersionsmitteln ein räumliches Netzwerk, wobei die Teilchen durch Neben- oder Hauptvalenzen an verschiedenen Punkten, sogenannten Haftpunkten aneinander heften. Werden die Zwischenräume zwischen den Teilchen mit Flüssigkeiten ausgefüllt, so liegt ein Lyogel, auch Gallerte genannt, vor. Ist die Flüssigkeit Wasser, werden die Gele oftmals auch als Hydrogele bezeichnet.

Im Rahmen der vorliegenden Erfindung ist es weiterhin üblicherweise vorgesehen, dass das Polysaccharid Wasser binden kann. Vorzugsweise ist es in diesem Zusammenhang vorgesehen, dass das Polysaccharid eine gewichtsbezogene Menge an Wasser binden kann, welche der 10.000- bis 15.000-fachen, vorzugsweise 100-bis 10.000-fachen, vorzugsweise 300- bis 8.000-fachen, Menge des eingesetzten Polysaccharids, bezogen auf die Massen an eingesetztem Polysaccharid, entspricht.

Polysaccharide mit hohem Wasserbindungsvermögen liegen in ihrer wässrigen Dispersion bzw. Lösung oftmals als Gele vor.

Im Rahmen der vorliegenden Erfindung ist es insbesondere vorgesehen, dass die wässrige Zusammensetzung eine stabile viskose Lösung oder Dispersion ist oder ein Hydrogel.

Polysaccharide mit hohem Wasserbindungsvermögen bilden mit Wasser oftmals viskose Zusammensetzungen. Im Rahmen der vorliegenden Erfindung wird es bevorzugt, wenn die wässrige Zusammensetzung eine dynamische Viskosität nach Brookfield bei 25 °C im Bereich von 0,5 bis 10 mPa·s, insbesondere 1 bis 5 mPa·s, vorzugsweise 1 bis 2 mPa·s, aufweist. Vorzugsweise wird die dynamische Viskosität mit einem Brookfield-Viskosimeter bei 25 °C mit Spindel 18 bei 100 Umdrehungen pro Minute gemessen.

Die erfindungsgemäße wässrige Zusammensetzung weist somit niedrige Viskositäten auf und kann somit insbesondere auch durch Sprühauftrag aufgebracht werden.

Unter dem Begriff "stabil" ist dabei insbesondere zu verstehen, dass sich die Zusammensetzung bei Lagerung über einen Zeitraum von mindestens 3 Monaten, vorzugsweise mindestens 6 Monaten, bevorzugt mindestens 12 Monaten, in ihren chemischen und physikalischen Eigenschaften nicht ändert. Gleichermaßen ist es im Rahmen der vorliegenden Erfindung vorzugsweise vorgesehen, dass die wässrige Zusammensetzung sich in ihren chemischen oder physikalischen Eigenschaften über einen Zeitraum im Bereich von 3 Monaten bis 5 Jahren, insbesondere 6 Monaten bis 3 Jahren, vorzugsweise 12 Monaten bis 2 Jahren, nicht ändert.

Im Rahmen der vorliegenden Erfindung ist es üblicherweise vorgesehen, dass das zur Herstellung des Komplexes eingesetzte Polysaccharid ein gewichtsmittleres Molekulargewicht (Molmasse) M_{w} im Bereich von 30 kDa bis 1.200 kDa, insbesondere im Bereich von 50 kDa bis 1.000 kDa, vorzugsweise im Bereich von 60 kDa bis 700 kDa, bevorzugt im Bereich von 70 kDa bis 500 kDa, besonders bevorzugt im Bereich von 80 kDa bis 300 kDa, ganz besonders bevorzugt im Bereich von 85 kDa bis 200 kDa, insbesondere bevorzugt 90 kDa bis 150 kDa, insbesondere besonders bevorzugt 90 kDa bis 120 kDa, aufweist.

Ganz besonders gute Ergebnisse werden in diesem Zusammenhang erhalten, wenn das Polysaccharid ein gewichtsmittleres Molekulargewicht (Molmasse) M_{w} im Bereich von 95 kDa bis 105 kDa aufweist. Polysaccharide in den zuvor beschriebenen Molekulargewichtsbereichen weisen einerseits hervorragende Eigenschaften im Bereich der Wasserbindung und Filmbildung aus, lassen sich jedoch andererseits gut in Wasser lösen bzw. dispergieren, so dass niederviskose Zusammensetzungen erhalten werden können.

Erfindungsgemäß kann das zahlenmittlere Molekulargewicht Mₙ und/oder das gewichtsmittlere Molekulargewicht M_{w} und/oder das zentrifugenmittlere Molekulargewicht M_{z} und/oder der Polydispersitätsindex (PDI) von oligomeren oder polymeren Verbindungen mittels Gelpermeationschromatographie (GPC), insbesondere gekoppelt mit Lichtstreudetektion (MALLS; *Multi Angle Laser Light Scattering*), und/oder gemäß DIN 55672-3:2016-03 bestimmt sein.

Im Rahmen der vorliegenden Erfindung werden besonders gute Ergebnisse erhalten, wenn das zur Herstellung des Komplexes eingesetzt Polysaccharid ausgewählt ist aus der Gruppe der Glykosaminoglykane und Polyglucosamine sowie deren Mischungen und Derivaten. Glykosaminoglykane sind linear aus sich wiederholenden Disacchariden aufgebaute, saure Polysaccharide, deren einzelne Disaccharideinheiten aus Uronsäure bestehen, welche 1-3-glycosidisch mit einem Aminozucker wie beispielsweise N-Acetylglucosamin verbunden sind. Die Disaccharideinheiten der Ketten sind dabei 1-4-glycosidisch verknüpft. Sie besitzen allesamt die Fähigkeit Wasser aufzunehmen und eine hohe Elastizität.

Polyglucosamine sind Derviate des Polyglucosamin, auch Chitosan genannt, welches aus β-1,4-glycosidisch verknüpften Glucosaminresten und gegebenenfalls N-Acetylglucosaminresten aufgebaut ist. Polyglucosamin bzw. Chitosan wird wie zuvor beschrieben aus Chitin durch Deacetylisierung, bspw. mit heißer Natronlauge oder enzymatisch, gewonnen. Die Deacetylisierung erfolgt dabei entweder vollständig oder auch nur teilweise, so dass Chitosan im Gegensatz zu Chitin nicht nur aus N-Acetylglucosaminen besteht, sondern Mischungen aus N-Acetylglucosaminen und Glucosaminen enthält. Ein zu 100 % deacetylisiertes Chitosan besteht folglich nur aus Glucosamineinheiten.

Polyglucosamin bzw. Chitosan und dessen Derivate werden für viele Anwendungen im Rahmen der vorliegenden Erfindung bevorzugt eingesetzt, da sie neben guten Wasserbindungs- und Filmbildungseigenschaften auch gewisse mikrobizide Eigenschaften aufweisen, wobei die mikrobiziden Eigenschaften nicht stark ausgeprägt sind. Es handelt sich oftmals eher um bakteriostatische oder fungistatische Eigenschaften, d.h. das Wachstum bzw. die Vermehrung von Bakterien oder Pilzen wird zwar gehemmt, jedoch nicht vollständig unterbunden. Chitosan ist allerdings ungiftig und kann daher für eine Vielzahl von Anwendungen eingesetzt werden.

Im Rahmen der vorliegenden Erfindung hat es sich bewährt, wenn das zur Herstellung des Komplexes eingesetzte Polysaccharid ausgewählt ist aus der Gruppe von Chitosan, Hyaluronsäure, Heparin, Chondroitinsulfat, Keratanasulfat sowie deren Mischungen und Derivaten, insbesondere Chitosan und Hyaluronsäure sowie deren Mischungen und Derivaten, vorzugsweise Chitosan und Chitosanderivaten. Mit den zuvor genannten Polysacchariden werden allesamt gute Ergebnisse erhalten, wobei speziell der Einsatz von Chitosan und Chitosanderivaten, nicht zuletzt aufgrund der im gewissen Maße vorhandenen mikrobiziden Eigenschaften, bevorzugt ist.

Was nun die Chitosanderivate anbelangt, welche im Rahmen der vorliegenden Erfindung verwendet werden können, so hat es sich bewährt, wenn das Chitosanderivat ausgewählt ist aus der Gruppe von Hydroxypropylchitosan, N-Octyl-N-trimethylchitosan, N-Octyl-O-glycolchitosan, N-[(2-hydroxy-3-trimethylammonium)-propyl]chitosanchlorid, Carboxymethylchitosan und deren Mischungen.

Gleichermaßen wird es im Rahmen der vorliegenden Erfindung bevorzugt, wenn das zur Herstellung des Komplexes eingesetzt Chitosan einen Deacetylierungsgrad von mindestens 75 %, insbesondere mindestens 80 %, vorzugsweise mindestens 85 %, bevorzugt mindestens 90 %, aufweist.

Was nun die Carbonsäure anbelangt, so kann diese im Rahmen der vorliegenden Erfindung aus einer Vielzahl geeigneter Carbonsäuren ausgewählt werden. Es hat sich jedoch bewährt, wenn die Carbonsäure ausgewählt ist aus Polycarbonsäuren, Hydroxycarbonsäuren und deren Mischungen. Im Hinblick auf die Polycarbonsäuren ist insbesondere die Verwendung von Di- und Tricarbonsäuren bevorzugt. Auch die Hydroxycarbonsäuren können mehrere Carbonsäurefunktionen aufweisen. Der Einsatz von Polycarbonsäuren und Hydroxycarbonsäuren ist bevorzugt, da diese eine erhöhte Hydrophilie aufweisen und die Löslichkeit des Komplexes in polaren Lösemitteln, insbesondere Wasser, erhöhen. Besonders gute Ergebnisse werden im Rahmen der vorliegenden Erfindung erhalten, wenn aromatische Polycarbonsäuren, aromatische Hydroxycarbonsäuren oder deren Mischungen eingesetzt werden.

Was nun die Hydroxycarbonsäuren anbelangt, die im Rahmen der vorliegenden Erfindung eingesetzt werden können, so können diese aus einer großen Auswahl geeigneter Hydroxycarbonsäuren ausgewählt werden. Es hat sich allerdings bewährt, wenn die zur Herstellung des Komplexes eingesetzte Hydroxycarbonsäure ausgewählt ist aus α-Hydroxycarbonsäuren, β-Hydroxycarbonsäuren und deren Mischungen, insbesondere aromatischen α-Hydroxycarbonsäuren, aromatischen β-Hydroxycarbonsäuren und deren Mischungen.

Obwohl prinzipiell eine Vielzahl von Hydroxycarbonsäuren geeignet ist, so hat es sich - wie zuvor ausgeführt - insbesondere als besonders vorteilhaft herausgestellt, wenn aromatische Hydroxycarbonsäuren eingesetzt werden. Diese verstärken zum einen eine gute Löslichkeit bzw. Dispergierfähigkeit des Komplexes und fördern zum anderen auch die desinfizierenden Eigenschaften des Komplexes, insbesondere falls Polysaccharide mit desinfizierenden Eigenschaften ausgewählt werden.

Gleichermaßen hat es sich im Rahmen der vorliegenden Erfindung bewährt, wenn die zur Herstellung des Komplexes eingesetzte Hydroxycarbonsäure ausgewählt ist aus der Gruppe von Apfelsäure, Zitronensäure, Isozitronensäure, Weinsäure, Glycolsäure, Milchsäure, Mandelsäure, Tatronsäure, β-Hydroxbuttersäure, Mevalonsäure, Gallussäure, Salicylsäure, 4-Hydroxybenzoesäure und deren Mischungen. Besonders gute Ergebnisse werden in diesem Zusammenhang erhalten, wenn die zur Herstellung des Komplexes eingesetzte Hydroxycarbonsäure ausgewählt ist aus Mandelsäure, Gallussäure, Salicylsäure, 4-Hydroxybenzoesäure und deren Mischungen. Auch hier werden wieder bevorzugt aromatische Hydroxycarbonsäuren eingesetzt, welche insbesondere bevorzugt werden, um positive Eigenschaften des Komplexes zu verstärken. Die Herstellung des Komplexes gelingt jedoch prinzipiell auch mit den anderen genannten Hydroxycarbonsäuren.

Insbesondere werden gute Ergebnisse erhalten, wenn die zur Herstellung des Komplexes eingesetzte Hydroxycarbonsäure ausgewählt ist aus der Gruppe von Mandelsäure, Salicylsäure und deren Mischungen, wobei die Verwendung von Salicylsäure ganz besonders bevorzugt ist.

Salicylsäure, auch 2-Hydroxybenzcarbonsäure bzw. 2-Hydroxybenzoesäure genannt, kommt in der Natur in einer Vielzahl von Pflanzen als Phytohormon vor. Salicylsäure wird insbesondere zur Herstellung von Arzneistoffen, wie beispielsweise Acetylsalicylsäure, verwendet, aber auch aufgrund seiner antimikrobiellen Wirkung als Konservierungsstoff in Kosmetika. Nachteilig an Salicylsäure ist eine reizende bzw. gewebeschädigende Wirkung auf Haut, Schleimhäute und Augen und eine Toxizität bei Gabe höherer Dosen. Wird Salicylsäure im Rahmen des erfindungsgemäß verwendeten Komplexes eingesetzt, so ist die Toxizität deutlich herabgesetzt, während insbesondere mikrobizide Wirkungen noch deutlich verstärkt werden.

Was die Polycarbonsäure anbelangt, so kann diese im Rahmen der vorliegenden Erfindung gleichfalls aus einer Vielzahl geeigneter Polycarbonsäuren ausgewählt werden. Vorzugsweise ist die Polycarbonsäure ausgewählt aus Tricarbonsäuren, Dicarbonsäuren und deren Mischungen. Besonders gute Ergebnisse werden im Rahmen der vorliegenden Erfindung erhalten, wenn die Polycarbonsäure ausgewählt aus aromatischen Tricarbonsäuren, aromatischen Dicarbonsäuren und deren Mischungen.

Gleichfalls werden sehr gute Ergebnisse erhalten, wenn die Polycarbonsäure ausgewählt ist aus Oxalsäure, Malonsäure, Bernsteinsäure, Glutarsäure, Adipinsäure, Pimelinsäure, Suberinsäure, Phthalsäure, Isophthalsäure, Terephthalsäure, Agaricinsäure, 1,2,3,-Propantricarbonsäure, Hemimellitsäure, Trimellitsäure, Trimesinsäure und deren Mischungen. Vorzugsweise ist die Polycarbonsäure ausgewählt ist aus Bernsteinsäure, Phthalsäure, Trimesinsäure und deren Mischungen, insbesondere Bernsteinsäure und Trimesinsäure.

Es hat sich darüber hinaus bewährt, wenn die Carbonsäure ausgewählt ist aus Mandelsäure, Gallussäure, Salicylsäure, 4-Hydroxybenzoesäure, Bernsteinsäure, Phthalsäure, Trimesinsäure und deren Mischungen, insbesondere Mandelsäure, Salicylsäure, 4-Hydroxybenzoesäure, Bernsteinsäure, Trimesinsäure und deren Mischungen. Ganz besonders bevorzugt ist die Carbonsäure, wie zuvor ausgeführt, Salicylsäure.

Was nun das Polyamin bzw. Guanid, insbesondere Biguanid, anbelangt, welches im Rahmen der vorliegenden Erfindung eingesetzt wird, so kann hier auch eine Vielzahl von Verbindungen ausgewählt werden. Allgemein wird es bevorzugt, wenn das Guanid ein Biguanid ist. Besonders gute Ergebnisse werden hierbei erhalten, wenn das Polyamid ein aliphatisches Polyamin ist und/oder wenn das Gaunid, insbesondere Biguanid, ein aliphatisches Guanid, insbesondere ein aliphatisches Biguanid, ist. Besonders bevorzugt werden im Rahmen der vorliegenden Erfindung Biguanide eingesetzt.

Wenn im Rahmen der vorliegenden Erfindung ein Polyamin eingesetzt wird, so hat es sich bewährt, wenn das Polyamin ausgewählt ist aus der Gruppe von Triaminen, Tetraminen, Pentaminen, Hexaminen, Polyethylenimin und deren Mischungen, insbesondere von aliphatischen Triaminen, aliphatischen Tetraminen, aliphatischen Pentaminen, aliphatischen Hexaminen, Polyethylenimin und deren Mischungen.

Besonders gute Ergebnisse werden in diesem Zusammenhang erhalten, wenn das Polyamin ausgewählt ist aus (C₂-C₁₈)Alkyldipropylentriamin, insbesondere Dodecyldipropylentriamin, Triethylentetramin, Tetraethylenpentamin und deren Mischungen.

Wenn im Rahmen der vorliegenden Erfindung ein Guanid, insbesondere Biguanid eingesetzt wird, so hat es sich bewährt, wenn das Guanid, insbesondere Biguanid ausgewählt ist aus der Gruppe von Polyhexamethylenbiguanid,_ Chlorhexidin, Alexidin, Dodecylguanidinmonohydrochlorid, (E)-1-(2-Chlor-1,3-thiazol-5-ylmethyl)-3-methyl-2-nitroguanidin (Clothianidin), Monohydrochlorid des Polymers aus N,N-1,6-Hexandiylbis[N-cyanoguanidin] (EINECS 240-032-4) und Hexamethylendiamin (EINECS 204-679-6), Oligo(2-(2-ethoxy)ethoxyethylguanidiniumchlorid), Poly(hexamethylendiaminguanidiniumchlorid) und deren Mischungen. Besonders gute Ergebnisse werden erhalten, wenn das Guanid ein Biguanid ist und ausgewählt ist aus der Gruppe von Polyhexamethylenbiguanid, Chlorhexidin, Alexidin und deren Mischungen, insbesondere Polyhexamethylenbiguanid, Alexidin und deren Mischungen. Besonders bevorzugt ist die Verwendung von Polyhexamethylenbiguanid. Im Rahmen der vorliegenden Erfindung hat es sich insbesondere gezeigt, dass besonders gute Ergebnisse erhalten werden, wenn das Polyamin bzw. das Biguanid multifunktionell ist und keine aromatischen Gruppen aufweist. Wenn Polyhexamethylenbiguanid verwendet wird, so hat es sich bewährt, wenn PHMB (1415; 4.7), d.h. Polyhexamethylenbiguanidhydrochlorid mit einer zahlenmittleren Molmasse (Mn) von 1415 und einem mittleren Polydispersitätenindex (PDI) von 4,7 eingesetzt wird.

Besonders gute Ergebnisse werden erhalten, wenn im Rahmen der vorliegenden Erfindung das Polyamin und/oder das Guanid, insbesondere Biguanid, bereits mikrobizide Wirkungen aufweist. In diesem Fall ist es möglich, mit äußerst geringen Mengen an Polyaminen bzw. Guanid, insbesondere Biguanid, sehr effiziente Desinfektionsmittel bereitzustellen, da die Wirkung des Polyamins bzw. Guanids, insbesondere Biguanids, durch die weiteren Komponenten der Zusammensetzung, insbesondere des Komplexes, verstärkt wird. Speziell bei Verwendung von Biguaniden, wie beispielsweise Polyhexamethylenbiguanid ist es insbesondere möglich, mit Mengen zu arbeiten, die für den Menschen ohne Weiteres verträglich und nicht toxisch sind, die jedoch in hervorragender Weise antimikrobiell wirken, und dies auch über längere Zeiträume, beispielsweise über mehrere Tage.

Was nun die Menge anbelangt, in welchen die Zusammensetzung den Komplex enthält, so kann diese in weiten Bereichen variieren. Es hat sich jedoch bewährt, wenn die Zusammensetzung den Komplex in Mengen von 0,05 bis 10 Gew.-%, insbesondere 0,1 bis 5 Gew.-%, vorzugsweise 0,2 bis 3 Gew.-%, bevorzugt 0,3 bis 2 Gew.-%, besonders bevorzugt 0,4 bis 1 Gew.-%, bezogen auf die Masse der Zusammensetzung, enthält.

Zusammensetzungen, welche den Komplex in nur geringen Mengen, d.h. hoher Verdünnung und somit geringer Viskosität enthalten, sind außerordentlich effektiv, sowohl im kosmetischen Bereich, insbesondere in Haar- und Hautpflegeprodukten, als auch als Desinfektionsmittel, insbesondere als filmbildendes Desinfektionsmittel mit Langzeitwirkung.

Im Rahmen der vorliegenden Erfindung hat es sich gleichermaßen bewährt, wenn die Zusammensetzung den Komplex in Mengen von höchstens 10 Gew.-%, insbesondere höchstens 5 Gew.-%, vorzugsweise höchstens 3 Gew.-%, bevorzugt höchstens 2 Gew.-%, besonders bevorzugt höchstens 1 Gew.-%, bezogen auf die Masse der Zusammensetzung, enthält.

Im Rahmen der vorliegenden Erfindung hat es sich weiterhin bewährt, wenn die Zusammensetzung das Polysaccharid in Mengen von höchstens 9 Gew.-%, insbesondere höchstens 4 Gew.-%, vorzugsweise höchstens 2 Gew.-%, bevorzugt höchstens 1 Gew.-%, besonders bevorzugt höchstens 0,8 Gew.-%, bezogen auf die Masse der Zusammensetzung, enthält.

Gleichermaßen werden gute Ergebnisse erhalten, wenn die Zusammensetzung das Polysaccharid in Mengen von 0,04 bis 9 Gew.-%, insbesondere 0,1 bis 4 Gew.- %, vorzugsweise 0,2 bis 2 Gew.-%, bevorzugt 0,3 bis 1 Gew.-%, besonders bevorzugt 0,4 bis 0,8 Gew.-%, bezogen auf die Masse der Zusammensetzung, enthält.

Was nun die Menge an Carbonsäure in der Zusammensetzung anbelangt, so kann diese in weiten Bereichen variieren, wobei es sich bewährt hat, wenn die Zusammensetzung die Hydroxycarbonsäure in Mengen von höchstens 9 Gew.-%, insbesondere höchstens 4 Gew.-%, vorzugsweise höchstens 2 Gew.-%, bevorzugt höchstens 1 Gew.-%, besonders bevorzugt höchstens 0,8 Gew.-%, bezogen auf die Masse der Zusammensetzung, enthält.

Weiterhin werden gute Ergebnisse erhalten, wenn die Zusammensetzung die Carbonsäure in Mengen von 0,02 bis 9 Gew.-%, insbesondere 0,02 bis 4 Gew.-%, vorzugsweise 0,1 bis 2 Gew.-%, bevorzugt 0,1 bis 1 Gew.-%, besonders bevorzugt 0,2 bis 0,8 Gew.-%, bezogen auf die Masse der Zusammensetzung, enthält.

Üblicherweise enthält die Zusammensetzung das Polyamin und/oder das Guanid, insbesondere Biguanid in Mengen von höchstens 1 Gew.-%, insbesondere höchstens 0,5 Gew.-%, vorzugsweise höchstens 0,3 Gew.-%, bevorzugt höchstens 0,2 Gew.-%, besonders bevorzugt höchstens 0,1 Gew.-%, bezogen auf die Masse der Zusammensetzung.

Weiterhin werden besonders gute Ergebnisse erhalten, wenn die Zusammensetzung das Polyamin und/oder das Guanid, insbesondere Biguanid in Mengen von 0,01 bis 1 Gew.-%, insbesondere 0,02 bis 0,5 Gew.-%, vorzugsweise 0,03 bis 0,3 Gew.-%, bevorzugt 0,04 bis 0,2 Gew.-%, besonders bevorzugt 0,05 bis 0,1 Gew.- %, bezogen auf die Masse der Zusammensetzung, enthält.

Besonders gute Ergebnisse werden im Rahmen der vorliegenden Erfindung erhalten, wenn die Zusammensetzung und/oder der Komplex ein gewichtsbezogenes Verhältnis von Polysaccharid zu Carbonsäure im Bereich von 1 : 2 bis 5 : 1, insbesondere 1 : 1,5 bis 3 : 1, vorzugsweise 1 : 1 bis 2,5 : 1, bevorzugt 1,1 : 1 bis 2 : 1, aufweist.

In den beschriebenen Mengenverhältnissen ist es insbesondere möglich, hocheffektive und stabile wässrige Zusammensetzungen zu erhalten.

Gemäß einer im Rahmen der vorliegenden Erfindung besonders bevorzugten Ausführungsform ist die wässrige Zusammensetzung wie folgt aufgebaut: Wässrige Zusammensetzung, insbesondere für Hygiene- und/oder Kosmetikanwendungen, wie zuvor beschrieben, wobei die Zusammensetzung einen Komplex aus
a) mindestens einem Polysaccharid, ausgewählt aus Chitosan und Hyaluronsäure sowie deren Mischungen und Derivaten,
b) mindestens einer Carbonsäure, ausgewählt aus Mandelsäure, Gallussäure, Salicylsäure, 4-Hydroxybenzoesäure, Bernsteinsäure, Phthalsäure, Trimesinsäure und deren Mischungen, und
c) mindestens einem aliphatischen Polyamin und/oder einem aliphatischen Guanid, insbesondere einem aliphatischen Biguanid, insbesondere Polyhexamethylenbiguanid, Alexidin und deren Mischungen, aufweist.

Zusammensetzungen mit dem zuvor genannten Komplex eignen sich insbesondere in hervorragender Weise sowohl für kosmetische Anwendungen als auch als Desinfektionsmittel.

Für diese besondere und bevorzugte Ausführungsform der vorliegenden Erfindung gelten alle Besonderheiten, Vorteile, Merkmale, insbesondere Mengenangaben der einzelnen Komponenten, welche zuvor allgemein im Zusammenhang mit anderen Ausführungsformen beschrieben wurden.

Darüber hinaus kann es im Rahmen der vorliegenden Erfindung vorgesehen sein, dass die Zusammensetzung weiterhin einen Alkohol enthält.

Alkohole können insbesondere beigefügt werden, um die Stabilität des Komplexes weiter zu erhöhen und insbesondere die Löslichkeit des Komplexes in dem Löse- bzw. Dispersionsmittel Wasser zu verbessern. Bevorzugt wird es im Rahmen der vorliegenden Erfindung jedoch, wenn die wässrige Zusammensetzung Wasser als einziges Löse- oder Dispersionsmittel enthält.

Falls die wässrige Zusammensetzung einen Alkohol enthält, so hat es sich bewährt, wenn die Zusammensetzung den Alkohol in Mengen von 1 bis 20 Gew.-%, insbesondere 2 bis 15 Gew.-%, vorzugsweise 5 bis 12 Gew.-%, bevorzugt, 6 bis 10 Gew.-%, bezogen auf die Zusammensetzung, enthält.

Darüber hinaus kann der Alkohol auch aus einer Vielzahl von bekannten Alkoholen ausgewählt sein. Üblicherweise ist der Alkohol jedoch ausgewählt aus der Gruppe von Methanol, Ethanol, 1-Propanol, 2-Propanol, 1-Butanol, 1-Pentanol, 1-Hexanol, Glykol, Polyalkoholen und deren Mischungen. Hierbei werden besonders gute Ergebnisse erhalten, wenn er Alkohol ausgewählt ist aus Ethanol, 1-Propanol, 2-Propanol, Glykol und deren Mischungen, vorzugsweise aus Ethanol, Glykol und deren Mischungen. Ganz besonders bevorzugt ist der Alkohol Ethanol.

Gemäß einer bevorzugten Ausführungsform der vorliegenden Erfindung ist es vorgesehen, dass die Zusammensetzung ein Tensid enthält.

Tenside sind grenzflächenaktive Substanzen, welche zum einen die Löslichkeit von Substanzen in einem Löse- oder Dispergiermittel erhöhen und zum anderen auch die Filmbildungseigenschaften und den Verlauf bzw. die Rheologie von Beschichtungsmassen und Filmen beeinflussen. Wenn die Zusammensetzung ein Tensid enthält, so hat es sich bewährt, wenn die Zusammensetzung das Tensid in Mengen von 1 bis 40 Gew.-%, insbesondere 5 bis 30 Gew.-%, vorzugsweise 10 bis 25 Gew.-%, bevorzugt, 15 bis 25 Gew.-%, bezogen auf die Masse der Zusammensetzung, enthält.

Durch die Zugabe von Tensiden kann die Stabilität des Komplexes sowie die Löslichkeit des Komplexes in dem Löse- bzw. Dispersionsmedium Wasser erhöht werden. Weiterhin können die Filmbildungseigenschaften positiv beeinflusst werden. Für kosmetische Anwendungen ist insbesondere relevant, dass Tenside die Schaumbildung und Schaumstruktur verbessern und so die Reinigungswirkung von Shampoos, Seifen und Waschlotionen verbessern.

Darüber hinaus hat es sich im Rahmen der vorliegenden Erfindung bewährt, wenn das Tensid eine quartäre Ammoniumverbindung, ein nicht-ionisches Tensid oder deren Mischung ist.

Nicht-ionische Tenside sind beispielsweise Alkohlethoxylate oder FettsäureAlkanolamide.

Vorzugsweise ist das Tensid ausgewählt aus quartären Ammoniumverbindungen, Alkohlethoxylaten, Fettsäure-Alkanolamiden und deren Mischungen.

Besonders gute Ergebnisse werden erhalten, wenn das Tensid eine quartäre Ammoniumverbindung ist. Quartäre Ammoniumverbindungen zeichnen sich insbesondere dadurch aus, dass sie oftmals antimikrobielle Eigenschaften aufweisen, wodurch die mikrobizide Wirkung der erfindungsgemäßen Zusammensetzung nochmals erhöht werden kann. Gleichermaßen sind sie jedoch auch als reinigende und schaumstabilisierende Tenside für die Haut- und Haarpflege einzusetzen und beeinflussen die Filmbildungseigenschaften es erfindungsgemäßen Komplexes positiv.

Besonders gute Ergebnisse werden in diesem Zusammenhang erhalten, wenn das Tensid ausgewählt ist aus Betain, Cholin, (C₂-C₂₀)-Alkyltrimethylammoniumsalzen, (C₂-C₂₀)-Arylalkyltrimethylammoniumsalzen und deren Mischungen und Derivaten.

Besonders gute Ergebnisse werden erhalten, wenn das Tensid ausgewählt ist aus (C₂-C₂₀)-Alkylbetainen, insbesondere Cocoamidopropylbetain, *N*,*N*,*N*-Trimethyl-1-hexadecanaminiumbromid, Benzalkoniumchlorid, Benzethoniumchlorid, Cetylalkoniumchlorid, Tetradecyltrimethylammoniumoxalat, Dimethyldioctadecylammoniumchlorid, Cetylpyridiniumchlorid, Benzyltrimethylammoniumchlorid, Cocoamidopropy-Ihydroxysultain und deren Mischungen.

Beste Ergebnisse werden im Rahmen der vorliegenden Erfindung jedoch erhalten, wenn das Tensid ausgewählt ist aus (C₂-C₂₀)-Alkylbetainen und deren Mischungen, insbesondere Cocoamidopropylbetain, ist. Bei den Betainderivaten handelt es sich um bewährte und oft genutzte Tenside, welche insbesondere für kosmetische Anwendungen hochverträglich sind und hervorragende Filmbildungs- und Desinfektionseigenschaften aufweisen.

Gemäß einer bevorzugten Ausführungsform der vorliegenden Erfindung ist die Zusammensetzung wie folgt aufgebaut:
Insbesondere weist die Zusammensetzung
I) einen Komplex aus
   a) mindestens einem Polysaccharid, ausgewählt aus Chitosan und Hyaluronsäure sowie deren Mischungen und Derivaten,
   b) mindestens einer Carbonsäure, ausgewählt aus Mandelsäure, Gallussäure, Salicylsäure, 4-Hydroxybenzoesäure, Bernsteinsäure, Phthalsäure, Trimesinsäure und deren Mischungen, und
   c) mindestens einem aliphatischen Polyamin und/oder einem aliphatischen Guanid, insbesondere einem aliphatischen Biguanid, insbesondere Polyhexamethylenbiguanid, Alexidin und deren Mischungen,
II) ggf. einen Alkohol und
III) ein Tensid, auf.

Derartige Zusammensetzungen sind sowohl zur Verwendung in oder als Kosmetika sowie in oder als Desinfektionsmittel geeignet. Noch weiter bevorzugt ist es im Rahmen der vorliegenden Erfindung jedoch, wenn die Zusammensetzung keinen Alkohol aufweist.

Eine besonders bevorzugte Zusammensetzung gemäß der vorliegenden Erfindung umfasst folgende Bestandteile:
I) einen Komplex aus
   a) mindestens einem Polysaccharid ausgewählt aus Chitosan und Hyaluronsäure sowie deren Mischungen und Derivaten,
   b) mindestens einer Carbonsäure,_ausgewählt aus Mandelsäure, Gallussäure, Salicylsäure, 4-Hydroxybenzoesäure, Bernsteinsäure, Phthalsäure, Trimesinsäure und deren Mischungen, und
   c) mindestens einem aliphatischen Polyamin und/oder einem aliphatischen Guanid, insbesondere einem aliphatischen Biguanid, insbesondere Polyhexamethylenbiguanid, Alexidin und deren Mischungen, und
II) ein Tensid.

Für diese besonders bevorzugten Ausführungsformen der vorliegenden Erfindung gelten alle Vorteile, Merkmale, Besonderheiten und bevorzugten Aspekte, welche zuvor im Zusammenhang mit anderen Ausführungsformen beschrieben wurden; dies gilt insbesondere für Mengenverhältnisse und die bevorzugte Auswahl der einzelnen Komponenten.

Bei der Zusammensetzung handelt es sich um eine wässrige Zusammensetzung. Die Mengen, in welcher die wässrige Zusammensetzung das Wasser enthält, kann dabei in Abhängigkeit der spezifischen Beschaffenheit der Zusammensetzung und der beabsichtigen Verwendung in weiten Bereichen variieren. Es hat sich jedoch bewährt, wenn die Zusammensetzung Wasser in Mengen von 45 bis 95 Gew.-%, insbesondere 50 bis 90 Gew.-%, vorzugsweise 60 bis 90 Gew.-%, bevorzugt 70 bis 85 Gew.-%, bezogen auf die Zusammensetzung, aufweist.

Gleichermaßen kann es vorgesehen sein, dass die Zusammensetzung Wasser in Mengen von mindestens 45 Gew.-%, insbesondere mindestens 50 Gew.-%, vorzugsweise mindestens 60 Gew.-%, bevorzugt mindestens 70 Gew.-%, bezogen auf die Zusammensetzung, aufweist.

Im Rahmen der vorliegenden Erfindung kann es weiterhin vorgesehen sein, dass die Zusammensetzung ein Additiv aufweist.

Falls die Zusammensetzung ein Additiv aufweist, so hat es sich bewährt, wenn die Zusammensetzung das Additiv in Mengen von 0,01 bis 10 Gew.-%, insbesondere 0,05 bis 5 Gew.-%, vorzugsweise 0,1 bis 2 Gew.-%, bevorzugt 0,2 bis 1 Gew.-%, bezogen auf die Zusammensetzung, aufweist.

Gleichermaßen kann es vorgesehen sein, dass die Zusammensetzung das Additiv in Mengen von höchstens 10 Gew.-%, insbesondere höchstens 5 Gew.-%, vorzugsweise höchstens 2 Gew.-%, bevorzugt höchstens 1 Gew.-%, bezogen auf die Zusammensetzung, aufweist.

Wenn die Zusammensetzung ein Additiv aufweist, so ist diese üblicherweise aus pH-Stellmitteln, Rheologiestellmitteln, Farbstoffen, Geruchsstoffen und deren Mischungen.

Was nun den pH-Wert der erfindungsgemäßen Zusammensetzung anbelangt, so kann diese in weiten Bereichen variieren. Üblicherweise weist die Zusammensetzung jedoch einen pH-Wert von 2 bis 6,6, insbesondere 2,5 bis 5, vorzugsweise 3 bis 4, bevorzugt 3,3 bis 3,8, auf.

Es zeigen die Figurendarstellungen gemäß
- Fig. 1: ein UV-VIS-Spektrum des erfindungsgemäß eingesetzten Komplexes und Vergleichsspektren;
- Fig. 2: eine Mikroskopieaufnahme in 100-facher Vergrößerung eines mit einer erfindungsgemäßen Zusammensetzung teilweise behandelten Lederstücks;
- Fig. 3: eine mikroskopische Aufnahme in 250-facher Vergrößerung eines mit der erfindungsgemäßen Zusammensetzung behandelten Lederstücks;
- Fig. 4A: eine mikroskopische Aufnahme in 100-facher Vergrößerung eines mit der erfindungsgemäßen Zusammensetzung teilweise behandelten Lederstücks;
- Fig. 4B: die Oberflächenrauigkeit des in Fig. 4A markierten Ausschnitts;
- Fig. 5: einen Vergleich zwischen dem erfindungsgemäßen Shampoo M 901 und der Referenzprobe PQ10;
- Fig. 6: Leitfähigkeitsmessungen an mit dem erfindungsgemäßen Shampoo M 901 behandeltem Haar sowie Referenzproben;
- Fig. 7: eine Konfokal-Mikroskopieaufnahme eines mit Standardshampoo behandelten Haares;
- Fig. 8: eine Konfokal-Mikroskopieaufnahme eines mit dem erfindungsgemäßen Shampoo M 901 behandelten Haares;
- Fig. 9: IR-Transmissionsspektren eines erfindungsgemäßen Komplexes und von Vergleichsproben;
- Fig. 10: eine Konfokal-Mikroskopieaufnahme einer Probe von Chitosan-Salicylat auf einem Objektträger;
- Fig. 11: eine Konfokal-Mikroskopieaufnahme einer Probe von Chitosan-Salicylat mit peripher vorliegendem PHMB auf einem Objektträger und
- Fig. 12: eine Konfokal-Mikroskopieaufnahme einer Probe eines erfindungsgemäßen Komplexes auf einem Objektträger.

Weiterer Gegenstand der vorliegenden Erfindung - gemäß einem **zweiten** Aspekt der vorliegenden Erfindung - ist die Verwendung einer zuvor genannten wässrigen Zusammensetzung in oder als Kosmetika, insbesondere in Produkten zur Haut- oder Haarpflege.

Die zuvor beschriebene wässrige Zusammensetzung eignet sich insbesondere als Grundkörper bzw. Basisformulierung für Cremes und Lotionen zur Pflege von Gesicht, Körper und Händen sowie für Shampoos und Spülungen zur Haarpflege.

Die erfindungsgemäße wässrige Zusammensetzung wirkt einerseits durch die Filmbildung pflegend und schützend und fördert die Wundheilung. Darüber hinaus wirkt sie auch desinfizierend.

Insbesondere in Produkten zur Haarpflege ist die Verwendung der erfindungsgemäßen Zusammensetzung besonders vorteilhaft, da zum einen die Strapazierfähigkeit des Haares insbesondere bei thermischer Belastung deutlich erhöht wird und andererseits die Kämmbarkeit verbessert wird. Darüber hinaus wirkt die erfindungsgemäße Zusammensetzung auch als Foam-Booster und erhöht die Reinigungswirkung in Shampoos, so dass auf mehrmalige Waschgänge verzichtet werden kann.

Aufgrund der hervorragenden Antifrizz-Eigenschaften ermöglicht die erfindungsgemäße Zusammensetzung, auf den Einsatz von Silikonen und Mikroplastik in Shampoos und Duschgelen zu verzichten.

Für weitere Einzelheiten in Bezug auf die erfindungsgemäße Verwendung einer wässrigen Zusammensetzung kann auf die obigen Ausführungen zu der erfindungsgemäßen wässrigen Zusammensetzung verwiesen werden, welche in Bezug auf die erfindungsgemäße Verwendung entsprechend gelten.

Wiederum weiterer Gegenstand der vorliegenden Erfindung - gemäß einem **dritten** Aspekt der vorliegenden Erfindung - ist die Verwendung einer zuvor beschriebenen wässrigen Zusammensetzung in oder als Desinfektionsmittel, insbesondere zur Desinfektion von Oberflächen aller Art.

Die erfindungsgemäße wässrige Zusammensetzung eignet sich auch als Grundkörper bzw. Basis zur Herstellung von Desinfektionsmitteln, wobei insbesondere hochverträgliche und nicht toxische Desinfektionsmittel erhalten werden, welche eine hohe Wirksamkeit aufweisen und neben einer spontanen, d.h. sofortigen Wirkung auch einen Langzeiteffekt durch die hervorragenden Filmbildungseigenschaften aufweisen.

Die Zusammensetzung kann dabei insbesondere zur Desinfizierung sämtlicher Oberflächen, beispielsweise in Einzelhandelsgeschäften aber auch in lebensmittelerzeugenden Betrieben, wie beispielsweise Fleischereien, Metzgereien, Bäckereien etc. verwendet werden.

Darüber hinaus können sogar Lebensmittel, wie beispielsweise Backwaren, unmittelbar mit der Zusammensetzung behandelt werden, ohne dass negative Auswirkungen sowohl auf das zu behandelnde Lebensmittel als auch im Verzehr zu befürchten sind.

Für weitere Einzelheiten zu der erfindungsgemäßen Verwendung der wässrigen Zusammensetzung kann auf die obigen Ausführungen zu den übrigen Erfindungsaspekten verwiesen werden, welche in Bezug auf die erfindungsgemäße Verwendung entsprechend gelten.

Wiederum weiterer Gegenstand der vorliegenden Erfindung - gemäß einem **vierten** Aspekt der vorliegenden Erfindung - ist eine kosmetische Zubereitung, welche die zuvor genannte wässrige Zusammensetzung enthält.

Besonders gute Ergebnisse werden dabei erhalten, wenn die kosmetische Zubereitung ausgewählt ist aus Produkten der Haut- und Haarpflege, insbesondere Seifen, Shampoos, Waschgelen, Cremes, Lotionen und dergleichen.

Für weitere Einzelheiten zu der kosmetischen Zubereitung kann auf die obigen Ausführungen zu den übrigen Erfindungsaspekten verwiesen werden, welche für die erfindungsgemäße kosmetische Zubereitung entsprechend gelten.

Wiederum weiterer Gegenstand der vorliegenden Erfindung - gemäß einem **fünften** Aspekt der vorliegenden Erfindung - ist ein Desinfektionsmittel, enthaltend oder bestehend aus der zuvor beschriebenen wässrigen Zusammensetzung.

Das erfindungsgemäße Desinfektionsmittel eignet sich in hervorragender Weise zur Desinfizierung von Oberflächen aller Art, insbesondere auch zur Desinfizierung von Lebensmitteln.

Für weitergehende Einzelheiten zu dem erfindungsgemäßen Desinfektionsmittel kann auf die obigen Ausführungen zu den übrigen Erfindungsaspekten verwiesen werden, welche in Bezug auf das erfindungsgemäße Desinfektionsmittel entsprechend gelten.

Wiederum weiterer Gegenstand der vorliegenden Erfindung - gemäß einem **sechsten** Aspekt der vorliegenden Erfindung - ist ein Verfahren zur Herstellung einer zuvor beschriebenen wässrigen Zusammensetzung, wobei in einem ersten Verfahrensschritt (i)
a) ein Polysaccharid, welches Monomereinheiten aus ggf. substituierten Aminozuckern, insbesondere Glucosaminen und/oder Galactosaminen, aufweist, und
b) eine Carbonsäure mit Wasser gemischt und unter Erwärmen gelöst und/oder fein dispergiert werden.

Vorzugsweise wird der Verfahrensschritt solange durchgeführt, bis sich eine Lösemittel feinteilige Dispersion gebildet hat.

Was nun die Erwärmung anbelangt, so kann die hierfür angewendeten Temperatur in weiten Bereichen variieren. Es hat sich jedoch bewährt, wenn die Mischung auf Temperaturen im Bereich von 40 bis 100 °C, insbesondere 50 bis 95 °C, vorzugsweise 60 bis 95 °C, bevorzugt 70 bis 90 °C, besonders bevorzugt 80 bis 90 °C, erwärmt wird. In diesem Temperaturebereichen ist eine schnelle und vollständige Lösung bzw. Dispergierung gesichert und eine chemische Verbindung zwischen dem Polysaccharid und der Säure entsteht.

Gleichermaßen werden gute Ergebnisse erhalten, wenn die Mischung während der Erwärmung durchmischt, insbesondere gerührt, wird. In diesem Zusammenhang hat es sich bewährt, wenn die einzelnen Komponenten der Mischung sehr vorsichtig durchmischt, insbesondere gerührt werden.

Üblicherweise schließt sich an den ersten Verfahrensschritt (i) ein zweiter Verfahrensschritt (ii) an. Hierbei hat es sich besonders bewährt, wenn in einem auf den ersten Verfahrensschritt (i) folgenden zweiten Verfahrensschritt (ii) die Lösung und/oder Dispersion gekühlt wird.

Die Kühlung erfolgt üblicherweise unmittelbar im Anschluss an Verfahrensschritt (i).

Besonders gute Ergebnisse werden in diesem Zusammenhang erzielt, wenn die Lösung und/oder Dispersion auf Temperaturen im Bereich von 30 bis 70 °C, insbesondere 40 bis 80 °C, vorzugsweise 45 bis 70 °C, bevorzugt 50 bis 60 °C, gekühlt wird. Die Abkühlgeschwindigkeit ist hierbei zweitrangig, es kann vielmehr auch passiv gekühlt werden, d.h. einfach durch Stehenlassen.

Weiterhin ist es im Rahmen der vorliegenden Erfindung weiterhin vorgesehen, dass im zweiten Verfahrensschritt (ii) die abgekühlte Lösung und/oder Dispersion mit einem Alkohol und/oder einem Tensid versetzt wird. Wie zuvor ausgeführt, ist die Verwendung eines Tensides im Rahmen der vorliegenden Erfindung bevorzugt.

Üblicherweise ist es im Rahmen der vorliegenden Erfindung vorgesehen, dass auf den zweiten Verfahrensschritt (ii) folgenden dritten Verfahrensschritt (iii) die Lösung und/oder Dispersion mit einem Polyamin und/oder einem Guanid, insbesondere einem Biguanid, versetzt wird. Im Rahmen der vorliegenden Erfindung ist es nicht zwingend notwendig, dass das Tensid vor dem Polyamid und/oder Guanid, insbesondere Biguanid, hinzugegeben wird, wenn die Zusammensetzung jedoch ein Tensid enthalten soll, so hat sich diese Reihenfolge bewährt, da deutlich einfacher ein stabiler Komplex erhalten werden kann.

Was die Temperaturen anbelangt, in welchen die Lösung oder Dispersion mit dem Polyamin und/oder Guanid, insbesondere Biguanid, versetzt wird, so hat es sich bewährt, wenn dies bei den gleichen Temperaturen geschieht, wie in Verfahrensschritt (ii).

Für weitergehende Einzelheiten zu den erfindungsgemäßen Verfahren kann auf die obigen Ausführungen zu den übrigen Erfindungsaspekten verwiesen werden, welche in Bezug auf das erfindungsgemäße Verfahren entsprechend gelten. Der Gegenstand der vorliegenden Erfindung wird nachfolgend in nicht beschränkender Weise durch die Ausführungsbeispiele eingehender beschrieben.

### Ausführungsbeispiele

### 1. Herstellung einer Beispielrezeptur

**Tabelle 1: Beispielrezeptur M228-2**

| Nr. | Verbindung | Gew.-% |
|---|---|---|
| 1 | Wasser | 79,11 |
| 2 | Chitosan | 0,50 |
| 3 | Salicylsäure | 0,30 |
| 4 | Cocamidopropylbetain | 20,00 |
| 5 | Polyexamethylenbiguanid | 0,09 |

Zur Herstellung einer erfindungsgemäßen Zusammensetzung werden Verbindungen 1, 2 und 3 gemäß Tabelle 1 bei 85 °C unter sachtem Rühren bis zur vollständigen Lösung bzw. feinteiligen Dispergierung der Feststoffe gemischt. Die Lösung bzw. Dispersion färbt sich aufgrund der Reaktion von Chitosan und Salicysäure beige-pink.

Die Mischung wird anschließend auf 55 °C gekühlt und unter vorsichtigem Rühren mit Verbindung 4 gemäß Tabelle 1 versetzt. Es bildet sich eine klare, homogene Mischung.

Die erhaltene Mischung wird bei 55 °C mit Verbindung 5 gemäß Tabelle 1 versetzt und bis zur vollständigen Lösung bzw. feinteiligen Dispersion gerührt.

Die so hergestellte erfindungsgemäße Zusammensetzung wird unter dem Kürzel M228-2 für die nachfolgenden Untersuchungen verwendet.

Die erhaltene Zusammensetzung weist folgende Parameter auf:

**Tabelle 2: Parameter der erfindungsgemäßen Beispielrezeptur**

| **Parameter** | **Prüfverfahren** | **Ergebnis** |
|---|---|---|
| Geruch | Sensorische Prüfung | geruchlos |
| Farbe | Sensorische Prüfung | Leicht trüb, beige-orange |
| Viskosität | Brookfield | 1,0 - 2,0 mPas |
| | - Spindel 18 | |
| | - 100 U/min | |
| | - 25 °C | |
| pH | pH-Meter | 3,3 - 3,8 |

Die erfindungsgemäße wässrige Zusammensetzung ist lagerstabil und problemlos mit wässrigen Lösungen und Emulsionen mischbar.

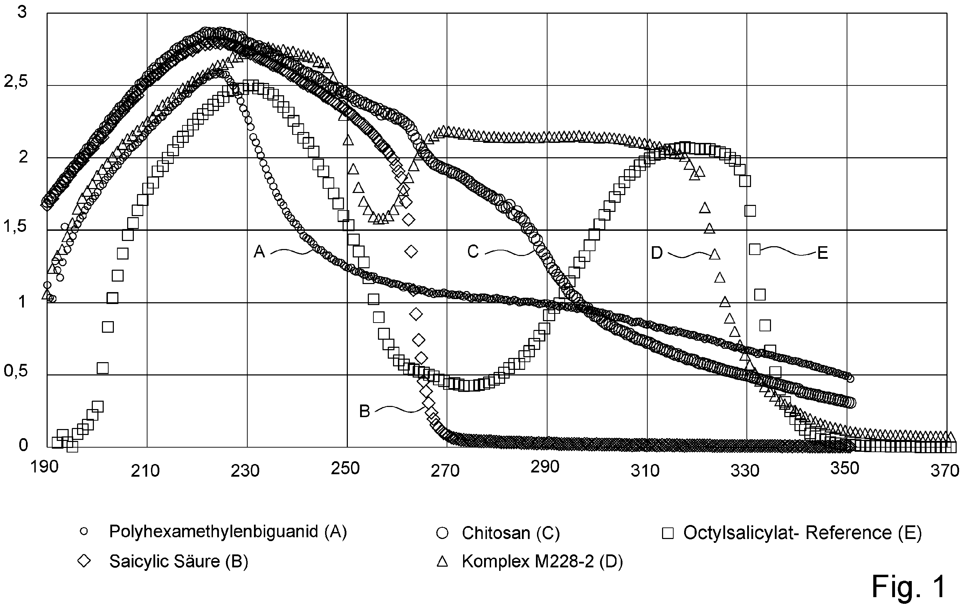

Die erfindungsgemäße wässrige Zusammensetzung wird darüber hinaus mittels UV-VIS-Spektroskopie untersucht. Die aufgenommenen Spektren sind in Fig. 1 dargestellt.

Zur Charakterisierung des gebildeten Komplexes wird die erfindungsgemäße wässrige Zusammensetzung M228-2 untersucht und mit geeigneten Substanzen verglichen. Als Vergleich werden die Einzelsubstanzen Chitosan, Salicylsäure, Polyhexamethylenbiguanid jeweils in einer Lösung von 20 % Cocoamidopropylbetain aufgenommen. Als Referenz wird ein weiteres Spektrum gegen Octylsalicylat in einer 20 %-igen Lösung von Cocoamidopropylbetain aufgenommen. Wie aus Fig. 1 ersichtlich ist, zeigt die erfindungsgemäße Zusammensetzung ein von den Einzelsubstanzen deutlich verschiedenes Absorptionsspektrum, welches auch nicht durch eine Überlagerung der Einzelspektren zu erklären ist, vielmehr muss davon ausgegangen werden, dass zwischen dem Chitosan, der Salicylsäure und dem Polyhexamethylenbiguanid intermolekulare Wechselwirkungen vorliegen, d.h. ein Komplex gebildet wird.

### 2. Filmbildung

Zur Demonstration der hervorragenden Filmbildungseigenschaften, welche sowohl in kosmetischen Produkten als auch in Desinfektionsmitteln von besonderer Bedeutung ist, werden nachfolgende Tests durchgeführt.

Als Model für menschliche Haut wird eine Lederfläche bereichsweise mit der erfindungsgemäßen wässrigen Zusammensetzung behandelt. Nach Trocknung beträgt die Schichtdicke auf den behandelten Stellen ca. 5 µm. Das beprobte Stück Leder wird anschließend mittels Konfokal-Mikroskopie untersucht.

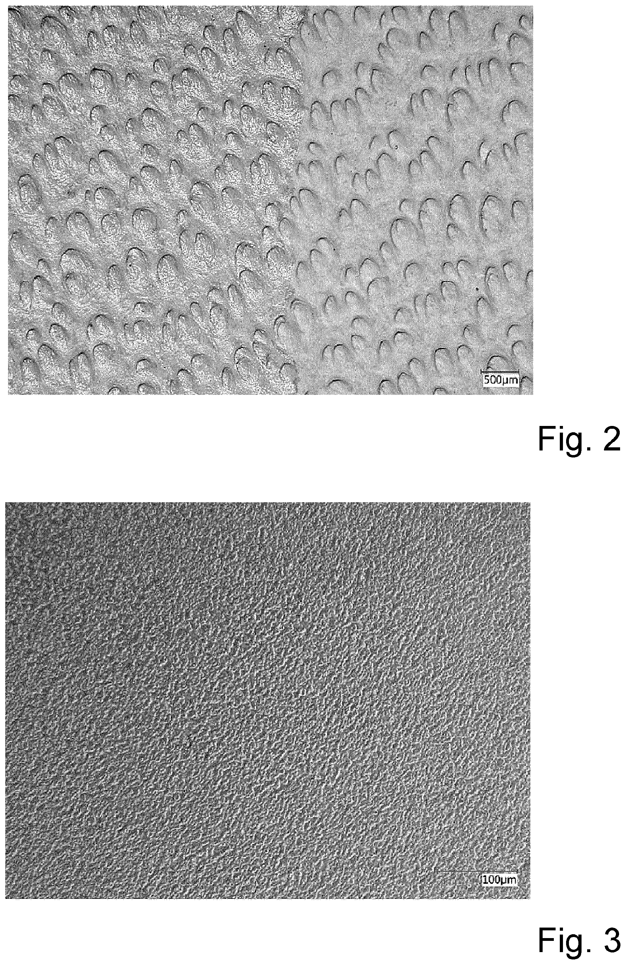

Es zeigt die Fig. 2 einen Teilbereich des beprobten Lederstücks in 100-facher Vergrößerung im Auflicht.

Fig. 2 zeigt auf der linken Seite die mit der erfindungsgemäßen Zusammensetzung beschichtete Oberfläche und auf der rechten Seite die unbeschichtete Oberfläche. Bereits bei dieser Vergrößerung ist ein scharfer Verlauf zwischen dem beschichteten und dem nicht beschichteten Teil im Auflicht zu erkennen. Der beschichtete Probenteil glänzt und lässt eine deutliche Filmbildung erkennen.

In Fig. 3 ist ein Ausschnitt aus dem beschichteten Bereich in 250-facher Vergrößerung im Auflicht gezeigt. Es zeigt sich, dass tatsächlich ein geschlossener Film vorliegt.

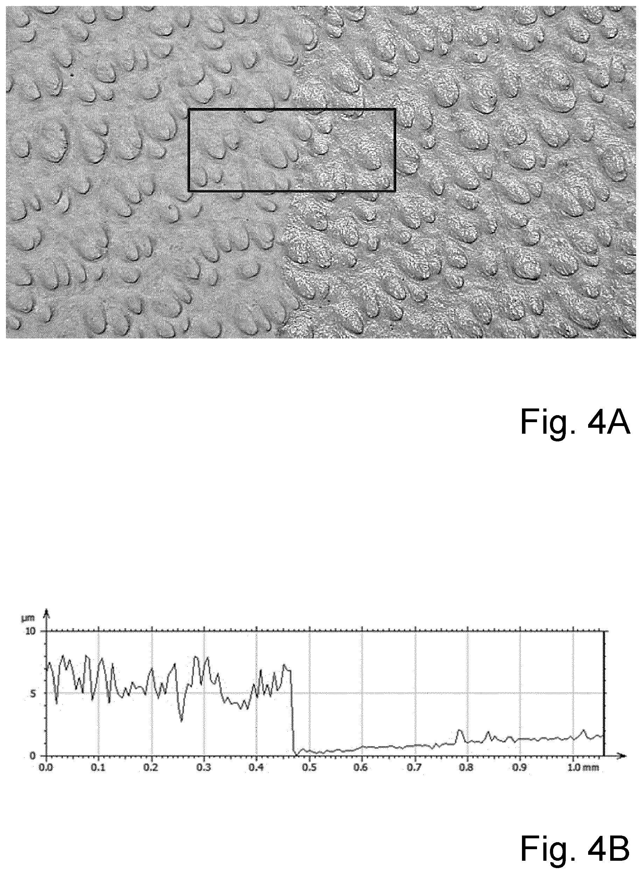

Fig. 4A zeigt wiederum in 100-facher Vergrößerung im Auflicht einen beschichteten Teil und einen unbeschichteten Teil der Probe, wobei in Fig. 4A der beschichtete Teil rechts aufgeführt ist, erkennbar an der leicht glänzenden Oberfläche, und der unbeschichtete Teil links dargestellt ist. In dem in Fig. 4A dargestellten Ausschnitt, welcher durch die schwarze Umrandung begrenzt wird, wurde eine Messung der Oberflächenrauigkeit durchgeführt. Die Breite des Ausschnitts beträgt 1,06 mm.

Die Oberflächenrauigkeit des Ausschnitts ist in Fig. 4B dargestellt. Links ist wiederum die Oberflächenrauigkeit des nicht beschichteten Teils und rechts die des mit der erfindungsgemäßen Zusammensetzung beschichteten Teils der Lederprobe wiedergegeben. Es zeigt sich, dass der beschichtete Teil eine sehr gleichmäßige Oberfläche aufweist, die Oberfläche geglättet wurde und einen Lotuseffekt aufweist, d.h. Schmutzanhaftungen deutlich reduziert werden.

### 3. Desinfektionstests

### 3.1 Desinfektionstests gemäß DIN/EN

Die nachfolgende Tabelle 3 gibt die nach DIN- bzw. EN-Norm durchgeführten Desinfektionstests an.

**Tabelle 3: Desinfektionstests nach DIN/EN**

| **Norm:** | **Beschreibung:** | **Ergebnis:** |
|---|---|---|
| DIN EN 1500 | Anwendungstests in vivo: Händedesinfektion-Keimreduktion | Bestanden |
| DIN EN 14348 | Chemische Desinfektionsmittel und Antiseptika - quantitativer Suspensionstest zur Bestimmung eines bakteriziden Effekts (Tuberkulose) von chemischen Desinfektionsmitteln in der Humanmedizin, einschließlich Instrumentendesinfektion | Bestanden |
| DIN EN 13727 | Chemische Desinfektionsmittel und Antiseptika - quantitativer Suspensionstest zur Bestimmung bakteriziden Effekts in der Humanmedizin | Bestanden |
| DIN EN 13624 | Chemische Desinfektionsmittel und Antiseptika - quantitativer Suspensionstest zur Bestimmung des fungiziden und hefeabtötenden Effekts in der Humanmedizin | Bestanden |
| DIN EN 14476 | Chemische Desinfektionsmittel und Antiseptika - quantitativer Suspensionstest zur Bestimmung des viruziden Effektes in der Humanmedizin | Bestanden |

### 3.1.1 Viruzide Eigenschaften

Nachfolgend werden detailliert die Ergebnisse der Desinfektionsprüfung nach DIN EN 14476, d.h. des quantitativen Suspensionstests zur Bestimmung des viruziden Effekts in der Humanmedizin ausführlicher dargelegt. In der nachfolgenden Tabelle 4 ist die Reduktion einiger pathogener Viren, welche gemäß DIN EN 14476 getestet wurde, wiedergegeben:

**Tabelle 4: Reduktion von Pathogenen**

| **Testvirus** | **Titerreduktion** | **Ergebnis¹** |
|---|---|---|
| Poliovirus | 4 log₁₀ | Bestanden |
| Adenovirus | 4 log₁₀ | Bestanden |
| Bovines/Murines | 4 log₁₀ | Bestanden |
| Parvovirus (ctD) | 4 log₁₀ | Bestanden |
| Murines | 4 log₁₀ | Bestanden |
| Norovirus | 4 log₁₀ | Bestanden |

| | | |
|---|---|---|
| ¹: Bestanden bei einer Titerreduktion von mindestens 4 log₁₀ gemäß DIN EN 14476 | | |

3.2 Weitere Tests zur mikrobiziden Wirkung

Zur Untersuchung der mikrobiziden Wirkung auf weitere Mikroorganismen wurden Nutriplate-Nährböden mit Keimen geimpft und anschließend inkubiert. Die Inkubation wird über 100 Stunden im Inkubator bei 35 °C durchgeführt. Anschließend erfolgt die Auszählung und visuelle Dokumentation der Nährbodenflächen.

Nachfolgend werden die Nährböden mit der erfindungsgemäßen Zusammensetzung M228-2 behandelt und die Nährböden 28 Tage bei 35 °C im Inkubator bebrütet, wobei alle sieben Tage erneut mit dem Pathogen geimpft wird.

In Tabelle 5 sind die untersuchten Mikroorganismen wiedergegeben.

**Tabelle 5: Untersuchte Mikroorganismen**

| **Mikroorganismus** | **Stamm/Reagenz** |
|---|---|
| Staphylococcus aureus | ATCC 6538 |
| Pseudomonas aeruginose | ATCC 9027 |
| Candida albicans | ATCC 10231 |
| Aspergillus brasiliensis (aspergillus niger) | ATCC 16404 |

In Tabelle 6 ist die Auswertung der Desinfektionsversuche wiedergegeben.

**Tabelle 6: Langzeitdesinfektionstest**

| Mikroorganismus | Impfung alle 7 Tage | Nach 100 h ohne Desinfektion | Nach 100 h nach Desinfektion | 7 Tage nach Desinfektion | 14 Tage nach Desinfektion | 28 Tage nach Desinfektion |
|---|---|---|---|---|---|---|
| Staphylococcus aureus | Gesättigte Impfkultur | Besiedlung vollflächig | 0 kbE¹ | 0 kbE | 1 kbE | 3 kbE |
| Pseudomonas aeruginose | Gesättigte Impfkultur | Besiedlung vollflächig | 0 kbE | 0 kbE | 1 kbE | 2 kbE |
| Candida albicans | Gesättigte Impfkultur | Besiedlung vollflächig | 0 kbE | 0 kbE | 0 kbE | 2 kbE |
| Aspergillus brasiliensis (aspergillus niger) | Gesättigte Impfkultur | Besiedlung vollflächig | 0 kbE | 0 kbE | 0 kbE | 5 kbE |

| | | | | | | |
|---|---|---|---|---|---|---|
| ¹: kolonienbildende Einheiten | | | | | | |

Es zeigt sich, dass die Behandlung mit der erfindungsgemäßen Zusammensetzung sowohl einerseits die Mikroorganismen sofort abtöten, als auch eine hervorragende Langzeitwirkung aufweist.

### 3.3 Feldversuch bzgl. der Langzeitwirkung

Zur Bestimmung der Langzeitdesinfektionswirkung unter alltäglichen Bedingungen werden Versuche in einem Einzelhandelsgeschäft durchgeführt. Hierzu werden drei Einkaufskörbe untersucht. Ein Korb wird nicht desinfiziert und bildet die Referenzprobe, ein weiterer Korb wird mit einem handelsüblichen Präparat desinfiziert - Dr. Schnell Desifor Quick Plus als Vergleichsprobe - und ein Korb wird mit der erfindungsgemäßen Zusammensetzung M228-2 behandelt.

Mit jedem Einkaufskorb führen jeweils drei unterschiedliche Kunden einen Probeeinkauf in dem Geschäft durch. Nach jedem Einkauf werden Dip-Slide-Proben von den Griffen der Einkaufskörbe genommen. Die Dip-Slides weisen einen TTC-Aga/Rose-Bengal-Aga-Nährboden auf und werden jeweils doppelseitig abgeklatscht. Die Dip-Slides werden 48 Stunden im Inkubator bei 35 °C bebrütet und im Anschluss erfolgt die Auszählung und visuelle Dokumentation der Nährbodenflächen. Die Ergebnisse sind in Tabelle 7 zusammengefasst.

**Tabelle 7: Desinfektionswirkung im Feldversuch**

| Probeeinkauf Nr. | M228-2 | Vergleich | Referenz |
|---|---|---|---|
| 1 | 0 kbE | > 50 kbE¹ | Besiedlung vollflächig |
| 3 | 0 kbE | > 10² kbE | Besiedlung vollflächig |

| | | | |
|---|---|---|---|
| ¹: kolonienbildende Einheiten | | | |

Der durchgeführte Test zeigt, dass die erfindungsgemäße Zusammensetzung M228-2 hervorragende Desinfektionseigenschaften aufweist, und die Wirkung lang anhält.

### 3.4 Wirksamkeit in der Bekämpfung schwarzen Schimmels in Bäckereien

Schimmel, insbesondere schwarzer Schimmel, aber auch Weißschimmel sind ein ernsthaftes Problem in industriellen Bäckereien, welches bislang nicht befriedigend gelöst werden konnte. Das Problem ist wohlbekannt und kann lediglich mit einem sehr hohen Hygienelevel halbwegs kontrolliert werden, welches jedoch in der Realität oftmals nicht erreicht wird. Es wird daher untersucht, inwieweit die erfindungsgemäße Zusammensetzung für diesen Zweck geeignet ist.

Zur Evaluierung werden die Oberfläche von 2 Metallblechen mittels Sprühauftrag mit der erfindungsgemäßen Zusammensetzung M228-2 behandelt. Ein Blech wird dabei einmalig behandelt und eines nach zehn Tagen ein zweites Mal. Als Kontrolle dient ein weiteres Blech, welches nicht behandelt wird. 10 Tage sowie 24 Tage nach der ersten Behandlung werden jeweils mittels Dip-Slide-Proben abgeklatscht und im Inkubator 48 Stunden bei 35 °C bebrütet. Die Dip-Slides besitzen einen TTC-Aga/Rose-Bengal-Aga-Nährboden und werden jeweils doppelseitig abgeklatscht. Die Auszählung und visuelle Dokumentation der kolonienbildenden Einheiten (kbEs) erfolgt stets nach 48 Stunden.

Die entsprechenden Ergebnisse sind in der nachfolgenden Tabelle 8 wiedergegeben:

**Tabelle 8: Reduktion von Schimmel auf Metallblechen**

| Blech-Nr. | Anwendungen | Anwendung Tag 0 | Probennahme nach 10 Tagen | | Desinfektion nach 10 Tagen | Probennahme Tag 24 | |
|---|---|---|---|---|---|---|---|
| | | | GKZ² | Schimmel | | GKZ | Schimmel |
| 1 | 1x | JA | <1*10³kbE¹ | 0 kbE | NEIN | <1*10²kbE | 2 kbE |
| 2 | 2x | JA | <1*10³kbE | 0 kbE | JA | <1*10kbE | 1 kbE |
| Referenz | - | NEIN | <1*10²kbE | SchimmelWachstum | NEIN | <1*10²kbE | Schimmelteppich |

| | | | | | | | |
|---|---|---|---|---|---|---|---|
| ¹: kolonienbildende Einheiten ²: Gesamtkeimzahl | | | | | | | |

### 3.5 Weitere Versuche zur Reduktion der Keimbelastung in industriellen Bäckereien

Mit den nachfolgenden Untersuchungen wird sowohl die Reduktion von Keimen, insbesondere von Schimmel, auf Abdecktüchern für Backbleche und auf Wände in Bäckereien untersucht.

Speziell die Kontamination von Tüchern, welche zum Abdecken von noch nicht fertigen Backwaren verwendet werden, ist ein großes Problem in industriellen Großbäckereien. Es wird nachfolgend untersucht, inwieweit es möglich ist, die Ausbreitung von Schimmel auf derartigen Tüchern zu verhindern bzw. selbst bei bereits vorhandenem Schimmelbefall den Schimmel abzutöten und eine weitere Verbreitung zu verhindern. Ziel ist es, durch geeignete Maßnahmen, den Reinigungszyklus in Bäckereien zu vergrößern.

Zur Durchführung des Versuches wird ein Backblech, welches mit einem Stofftuch, das einen visuell wahrnehmbaren anfänglichen Schimmelbefall aufweist, in einem Fermenationsraum einer industriellen Großbäckerei gesondert gelagert und über eine Woche im Hinblick auf die Keimbelastung untersucht. Zur Durchführung des Versuches wird eine offensichtlich mit Schimmel belastete Stelle mit einer Abklatschplatte abgeklatscht. Die Abklatschplatte enthält eine Caseinpepton-Sojamehlpepton-Agar-Nährboden und wird nach dem Abklatschen 48 Stunden im Inkubator bei 35 °C bebrütet.

Im Anschluss erfolgt die Auszählung visueller Dokumentationen der Nährbodenflächen.

Nach der ersten Probennahme wird die entsprechende Stelle mit der erfindungsgemäßen Zusammensetzung M228-2 behandelt und erneut eine Probe genommen. Nach einer Woche wird nochmals eine Probe genommen.

**Tabelle 9: Reduktion des Gesamtkeimzahl und des Schimmelbefalls auf Stoffabdeckungen für Backbleche**

| Behandlung | Tag 0 | | Tag 7 | |
|---|---|---|---|---|
| | GKZ² | Schimmel | GKZ | Schimmel |
| M228 | 0 | - | 0 | - |
| Referenz | 10⁵ kbE¹ | Wachstum | >1* 10⁵kbE | Großflächiges Schimmelwachstum |

| | | | | |
|---|---|---|---|---|
| ¹: kolonienbildende Einheiten ²: Gesamtkeimzahl | | | | |

Vergleichbare Versuche werden auch an einer Wand des Fermentationsraumes durchgeführt:
Hierzu werden Proben vor und unmittelbar nach der Desinfektion genommen sowie eine Woche nach der Desinfektion und zwei Wochen nach der Desinfektion. Die entsprechenden Ergebnisse sind in Tabelle 10 zusammengefasst.

**Tabelle 10: Gesamtkeimzahl und Schimmelbelastung an den Wänden des Fermentationsraumes**

| Zeitpunkt | Keimbelastung | |
|---|---|---|
| | GKZ² | Schimmel |
| Vor Desinfektion (Tag 0) | > 1*10⁴ kbE¹ | < 10 kbE |
| Nach Desinfektion (Tag 0) | > 10 kbE | < 10 kbE |
| Tag 7 | > 10 kbE | > 10 kbE |
| Tag 14 | > 10⁴ kbE | < 10 kbE |

| | | |
|---|---|---|
| ¹: kolonienbildende Einheiten ²: Gesamtkeimzahl | | |

Aus den obigen Daten ergibt sich, dass sowohl die gesamt Keimbelastung als auch der Schimmelbefall gestoppt wird und auch eine Neubesiedlung über einen Zeitraum von mindestens 1 Woche verhindert wird.

### 3.6 Wirksamkeit gegen Weißschimmel

Bei Backwaren, insbesondere industriell gefertigten und verpackten Backwaren ist oftmals zu beobachten, dass die Produkte in der Verpackung Weißschimmel ausbilden. Dies stellt zwar kein Gesundheitsrisiko für den Verbraucher dar, führt jedoch dazu, dass die Produkte vom Verbraucher nicht mehr angenommen werden.

Zur Evaluierung der Wirksamkeit der erfindungsgemäßen Zusammensetzung gegen weißen Schimmel werden Aufbackbrötchen, welche in der Packung Weißschimmel ausgebildet hatten, untersucht.

Ein Brötchen wird mit der erfindungsgemäßen Zusammensetzung M228-2 besprüht, während ein anderes, welches gleichfalls Weißschimmel ausgebildet hat, nicht behandelt wird. Ein weiteres Brötchen, welches keinerlei Ausbildung von Weißschimmel aufwies, wird als weitere Referenz untersucht. Die Produkte werden für 12 Stunden bei 35 °C und 80 % Luftfeuchtigkeit gelagert und anschließend mittels Dip-Slides untersucht.

Hierbei wurden sowohl Dip-Slides wie oben angegeben beprobt und anschließend im Inkubator bei 35 °C für 48 Stunden bebrütet. Außerdem wurde noch jeweils eine Abklatschplatte beprobt und gleichfalls im Inkubator bebrütet. Die Ergebnisse sind in der nachfolgenden Tabelle 11 zusammengefasst.

**Tabelle 11: Zusammenfassung der Versuche zur Reduktion von Weißschimmel**

| Probe | Schimmelbelastung (Dip Slide) | GKZ² (Dip-Slides) | GKZ (Abklatschplatte) |
|---|---|---|---|
| Produkt ohne Weißschimmel - unbehandelt | 10² kbE¹ | 10³ kbE | 10² kbE |
| Produkt mit Weißschimmel - unbehandelt | Besiedelung vollflächig | Teilweise Besiedelung | Teilweise Besiedelung |
| Produkt mit Weißschimmel - behandelt | 0 kbE | 0 kbE | < 10² kbE |

| | | | |
|---|---|---|---|
| ¹: kolonienbildende Einheiten ²: Gesamtkeimzahl | | | |

Es zeigt sich, dass die erfindungsgemäße Zusammensetzung M228-2 eine hohe Wirksamkeit sowohl gegenüber einer Vielzahl von Bakterien als auch gegenüber verschiedenen Schimmelarten aufweist. Die erfindungsgemäße Zusammensetzung weist dabei sowohl eine hohe Wirksamkeit bei der spontanen Wirkung auf als auch bei der Langzeitwirkung.

### 4. Kosmetische Anwendungen

### 4.1 Reduktion des Wasserverlustes der Haut

Die erfindungsgemäße Zusammensetzung M228-2 wird auf ihre Hautverträglichkeit hin untersucht.

Zu diesem Zweck wird der transepidermale Wasserverlust der Haut gemessen. Die Verdunstungsrate von Wasser aus der Haut kann mithilfe einer entsprechenden Tewameter-Sonde bestimmt werden. Auf diese Weise ist es möglich, Informationen über den Wasserverlust der Haut in Reaktion auf Umwelteinflüsse, beispielsweise hautreizende Stoffe, wie Desinfektionsmittel zu bestimmen. Es ist daher möglich, eine Aussage darüber zu tätigen, wie ein Inhaltsstoff bzw. eine Zusammensetzung das Feuchtigkeitsgleichgewicht der Haut beeinflusst. Somit kann abgeschätzt werden, ob ein Produkt einen pflegenden oder einen irritierenden Einfluss auf die Hautmembran aufweist. Die gemessenen Werte zeigen die Verdunstungsrate in g/h/m².

Die Bestimmung des Feuchtigkeitsverlusts der Haut beruht darauf, dass über die Haut ständig Wasser verdunstet, der sogenannte transepidermale Wasserverlust (transepidermal water loss - TEWL); dieser ist wichtig und stellt einen normalen Metabolismus der Haut dar.

Wird jedoch die Barrierefunktion der Haut beschädigt oder angegriffen, so zeigt sich dieses unmittelbar in einem erhöhten transepidermalen Wasserverlust. Dies gilt bereits für kleinste Verletzungen bzw. Schädigungen, welche mit dem bloßen Auge nicht zu erkennen sind.

Die Tewameter-Sonde misst die Wasserverdunstungsrate der Haut indirekt, mithilfe von zwei Sensoren, nämlich Sensoren zur Bestimmung der Temperatur und der relativen Luftfeuchtigkeit, in einem hohlen Zylinder. Diese Methode ist die einzige, die den Wert des transepidermalen Wasserverlustes messen kann ohne das Mikroklima auf der Haut zu verändern.

Die folgenden Messungen werden mit einem Tewameter TM300 durchgeführt. Insgesamt werden 35 Probanden untersucht und es werden Messungen vor und nach Anwendung des Desinfektionsmittels in Abhängigkeit der Zeit durchgeführt. Das Desinfektionsmittel wird dabei auf dem Unterarm angewandt.

Es werden jeweils drei unterschiedliche Messungen durchgeführt, nämlich zum einen mit der erfindungsgemäßen Zusammensetzung M228-2, einem Vergleichsmedium, nämlich Dr. Schnell Händedesinfektion (Vergleich), sowie einer Referenzprobe, dem WHO-desinfection-variant 1. Die Ergebnisse sind in der nachfolgenden Tabelle 12 zusammengefasst.

**Tabelle 12: Transepidermaler Wasserverlust**

| **Parameter** | **M 228-2 [g/h/m²]** | **Vergleich [g/h/m²]** | **Referenz [g/h/m²]** |
|---|---|---|---|
| Vor Anwendung | 13,1 | 13,1 | 13,1 |
| Unmittelbar nach Anwendung | 13,6 | 30,4 | 34,7 |
| 1 Stunde nach Anwendung | 13,5 | 23,2 | 25,1 |

Wie in Tabelle 12 ersichtlich ist, hat die Behandlung mit Desinfektionsmitteln oftmals einen erheblichen Einfluss auf den transepidermalen Wasserverlust der Haut. Es zeigt sich, dass sowohl bei der Vergleichsprobe als auch bei der Referenzprobe der transepidermale Wasserverlust stark ansteigt und selbst nach einer Stunde gegenüber dem Ausgangswert nahezu verdoppelt ist. Die erfindungsgemäße Zusammensetzung M 228-2 zeigt hingegen eine nahezu konstanten Wasserverlust, d.h. eine Reizung und Schädigung der Haut wird vermieden.

Die erfindungsgemäße Zusammensetzung eignet sich somit einerseits in hervorragender Weise als Handdesinfektionsmittel sowie andererseits auch in kosmetischen Zusammensetzungen.

### 4.2 Haarkonditionierung

Es wird ein Shampoo, welches die erfindungsgemäße Zusammensetzung (M 901) enthält mit einer Referenzrezeptur verglichen. Die Rezepturen unterscheiden sich lediglich darin, dass das Chitosan-Salicylat aus der erfindungsgemäßen Rezeptur M901 gegen Polyquaternium-10 ausgetauscht wurde, ein bekanntes filmbildendes quartäres Ammoniumsalz.

**Tabelle 13: Erfindungsgemäße Rezeptur M901**

| **Rohstoff** | | **Zusammensetzung** | |
|---|---|---|---|
| **INCI:** | **Anteil aktiver Rohstoff [Gew.-%]** | **Anteil aktiver Rohstoff [Gew.-%]** | **Anteil Rohstoff-Formulierung [Gew.-%]** |
| Chitosan Salicylate | 13,0 | 1,3 | **10** |
| Polysorbate 20 | 100,0 | 5,00 | **5,00** |
| Glycerin | 99,5 | 19,90 | **20,00** |
| Citrus Aurantium Dulcis Peel Cera | 100,0 | 0,50 | **0,50** |
| Aqua | 0,0 | 0,00 | **ad 100** |
| Polyhexamethylenbiguanide | 100 | 0,01 | **0,01** |
| Cymbopogon Schoenanthus Oil | 100 | 0,10 | **0,10** |
| Coco-Glucoside | 50,0 | 12,50 | **25,00** |

**Tabelle 14: Referenz-Rezeptur PQ10**

| **Rohstoff** | | **Zusammensetzung** | |
|---|---|---|---|
| **INCI:** | **Anteil aktiver Rohstoff [Gew.-%]** | **Anteil aktiver Rohstoff [Gew.-%]** | **Anteil Rohstoff-Formulierung [Gew.-%]** |
| POLYQUATERNIUM-10 | 100 | 1,3 | **1,3** |
| Polysorbate 20 | 100,0 | 5,00 | **5,00** |
| Glycerin | 99,5 | 19,90 | **20,00** |
| Citrus Aurantium Dulcis Peel Cera | 100,0 | 0,50 | **0,50** |
| Aqua | 0,0 | 0,00 | **ad 100** |
| Polymehamethylbiquanide | 100 | 0,01 | **0,01** |
| Cymbopogon Schoenanthus Oil | 100 | 0,10 | **0,10** |
| Coco-Glucoside | 50,0 | 12,50 | **25,00** |

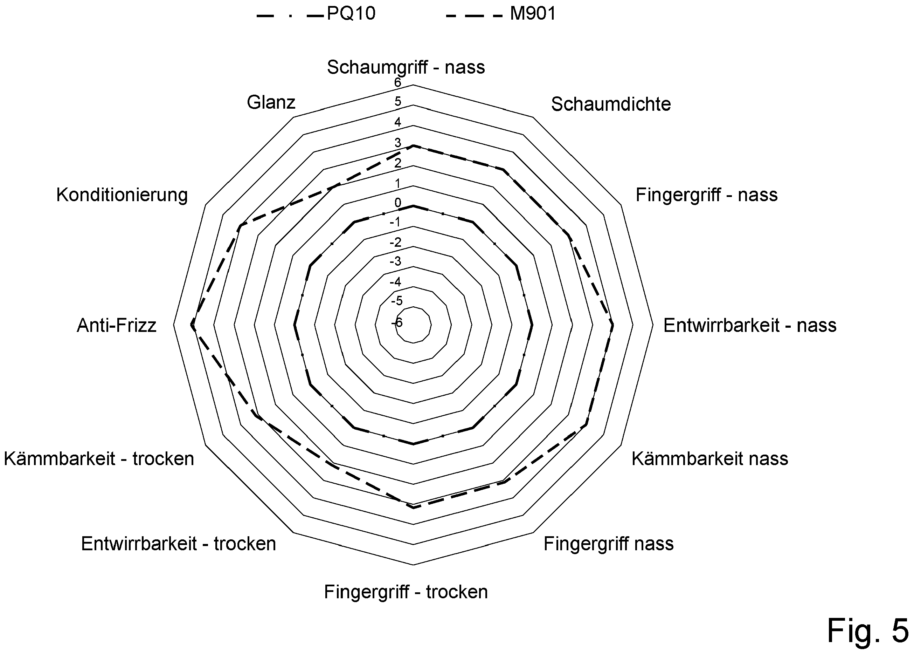

Die erfindungsgemäße Zusammensetzung sowie die Referenzzusammensetzung werden jeweils an 10 unterschiedlichen Probanden getestet. Die Performance der Shampoos werden dabei miteinander verglichen. Die Ergebnisse sind in Fig. 5 dargestellt, wobei das Referenzshampoo PQ10 überall den Wert 0 zugeordnet bekommt. Es zeigt sich, dass das erfindungsgemäße Shampoo M901 in allen Belangen deutlich überlegen ist.

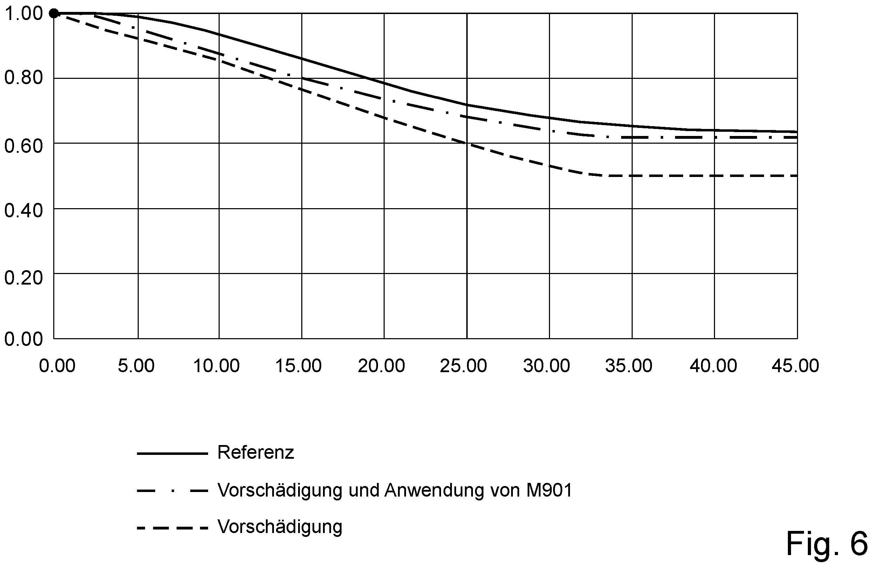

Darüber hinaus wird auch das Wasserbindungspotential von mit dem erfindungsgemäßen Shampoo M901 behandeltem Haar bestimmt. Hierzu werden drei Proben miteinander verglichen, nämlich vollständig unbehandeltes Haar als Referenzprobe, vorgeschädigtes Haar als Vergleich und vorgeschädigtes Haar, das mit dem erfindungsgemäßen Shampoo behandelt wurde. Die Vorschädigung wird durch eine Standardblondierung herbeigeführt. Das Wasserbindungspotential wird durch Leitfähigkeitsmessung bestimmt. Die Ergebnisse der Leitfähigkeitsmessung sind in Fig. 6 dargestellt. Es zeigt sich in Fig. 6 ganz eindeutig, dass durch die Anwendung des erfindungsgemäßen Shampoos M901 eine signifikante Restrukturierung erfolgt. Die Leitfähigkeit nähert sich stark der Referenzprobe, d.h. dem nicht vorgeschädigten Haar, an.

Darüber hinaus wird auch der Einfluss der erfindungsgemäßen Zusammensetzung auf die Farbstabilität von gefärbtem Haar untersucht. Hierzu werden zwei Probandinnen mit frisch gefärbten Haaren untersucht. Bei der Vergleichsprobe werden die Haare mit einem Standardshampoo gewaschen, während die anderen Probandinnen, ihre Haare mit dem erfindungsgemäßen Shampoo M 901 wäscht. Die Haare werden jeweils 15 mal gewaschen und die Farbe des Haars vor und nachher anhand der LAB-Werte bestimmt. Die Ergebnisse sind in Tabelle 15 wiedergegeben.

**Tabelle 15: Farbstabilisierung durch Anwendung von M901**

| Probe | Beschreibung | L* | a* | b* |
|---|---|---|---|---|
| M901 | unmittelbar nach Färbung | 35,62 | 4,35 | 3,30 |
| | Nach 15 Haarwäschen | 39,55 | 5,32 | 3,69 |
| Referenz | Unmittelbar nach Färbung | 35,96 | 4,35 | 3,30 |
| | Nach 15 Haarwäschen | 27,72 | 3,69 | 7,82 |

Wie Daten in Tabelle 15 zeigen, wird durch die Anwendung der erfindungsgemäßen Zusammensetzung M901 eine deutliche Farbstabilisierung erreicht.

Weiterhin wird die Konditionierung von vorgeschädigtem Haar (Standard-Blondier-Vorschädigung) anhand der oben aufgeführten Shampoos M901 und der Referenz PQ10 verglichen. Hierzu werden einzelne Haare nach Behandlung mit PQ10 bzw. M901 mittels Konfokal-Mikroskopie untersucht.

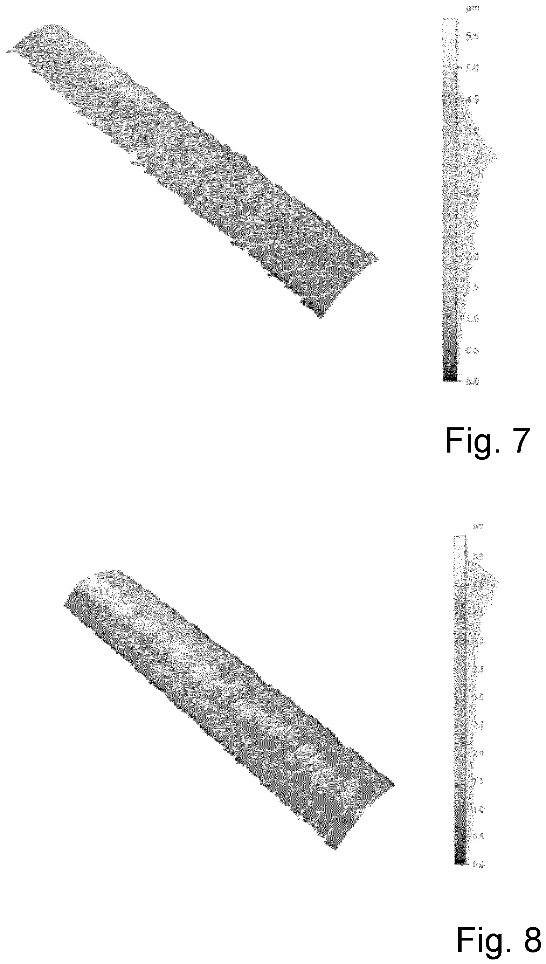

Die Ergebnisse sind in Fig. 7 und Fig. 8 dargestellt.

Fig. 7 zeigt dabei eine Konfokal-Mikroskopie-Aufnahme eines mit der Referenz PQ10 behandelten Haares und Fig. 8 zeigt eine Konfokal-Mikroskopie-Aufnahme eines mit dem erfindungsgemäßen Shampoo M901 behandelten Haares. Es zeigt sich, dass mit dem erfindungsgemäßen Shampoo M901 ein deutlich dickerer Schichtaufbau auf dem vorgeschädigten Haar gelingt. Das Verteilungsmaximum ist hier bei 5 µm anzusetzen, während mit dem normalen Shampoo ein Aufbau von ca. 3,5 µm erfolgt.

Insgesamt ist auch eine deutlich anliegendere Schuppenschicht bei der M901-Applikation zu erkennen. Die Strähne ist als ausladender konditioniert anzusehen.

### 5. Weitere Charakterisierung des erfindungsgemäßen Komplexes

Zum weiteren Nachweis des erfindungsgemäßen Komplexes werden Lösungen von Chitosan-Salicylat, Chitosan-Salicylat in Gegenwart von Polyhexamethylenbiguanid (PHMB) und eines erfindungsgemäßen Komplexes aus Chitosan, Salicysäure und Polyhexamethylenbiguanid (PHMB) hergestellt. Die Lösungen werden anschließend IR-spektroskopisch untersucht und mittels Konfokalmikroskopie auf ihre Filmbildungseigenschaften hin untersucht.

### 5.1 Herstellung einer Lösung von Chitosan-Salicylat

Es wird eine wässrige Lösung mit folgenden Bestandteilen hergestellt:

| | |
|---|---|
| 1,5 Gew.-% | Chitosan |
| 1 Gew.-% | Salicylsäure |

Die Edukte werden bei 70 °C umgesetzt, und zwar gesättigt als Funktion der Zeit bis zur vollständigen Umsetzung. Hierzu wurden wegorientiert IR-Proben während der Umsetzung untersucht und gleichsam die Farbzahl (nach Gardner) bestimmt, bis auch hier analytisch eine Wertesättigung eintritt.

Als Produkt wird bei nahezu vollständiger Umsetzung Chitosan-Salicylat erhalten.

### 5.2 Herstellung einer Lösung von Chitosan-Salicylat und anschließende Zugabe von PHMB

Es wird eine wässrige Lösung mit folgenden Bestandteilen hergestellt:

| | |
|---|---|
| 1,4 Gew.-% | Chitosan |
| 1 Gew.-% | Salicylsäure |
| 0,1 Gew.-% | PHMB |

Chitosan und Salicylsäure werden bei 70 °C umgesetzt, und zwar gesättigt als Funktion der Zeit bis zur vollständigen Umsetzung. Hierzu werden wegorientiert IR-Proben während der Umsetzung untersucht und gleichsam die Farbzahl (nach Gardner) bestimmt, bis auch hier analytisch eine Wertesättigung eintritt. Im Anschluss wird PHMB zugegeben und bei Raumtemperatur weitere 10 Stunden gerührt.

Als Produkt wird bei nahezu vollständiger Umsetzung Chitosan-Salicylat mit peripher vorliegendem PHMB erhalten.

### 5.3 Herstellung eines erfindungsgemäßen Komplexes

Es wird eine wässrige Lösung mit folgenden Bestandteilen hergestellt:

| | |
|---|---|
| 1,4 Gew.-% | Chitosan |
| 1 Gew.-% | Salicylsäure |
| 0,1 Gew.-% | PHMB |

Chitosan und Salicylsäure sowie PHMB werden bei 70° C umgesetzt, und zwar gesättigt als Funktion der Zeit bis zur vollständigen Umsetzung. Hierzu werden wegorientiert IR-Proben während der Umsetzung untersucht und gleichsam die Farbzahl (nach Gardner) bestimmt, bis auch hier analytisch eine Wertesättigung eintritt.

Als Produkt wird ein Chitosan-Guanidin-Salicylat (erfindungsgemäßer Komplex) bei nahezu vollständiger Umsetzung erhalten.

### 5.4 IR-spektroskopische Untersuchungen

Proben der unter 5.1 bis 5.3 hergestellten Lösungen werden IR-spektroskopisch untersucht, indem Transmissionsspektren der Lösungen aufgenommen werden.

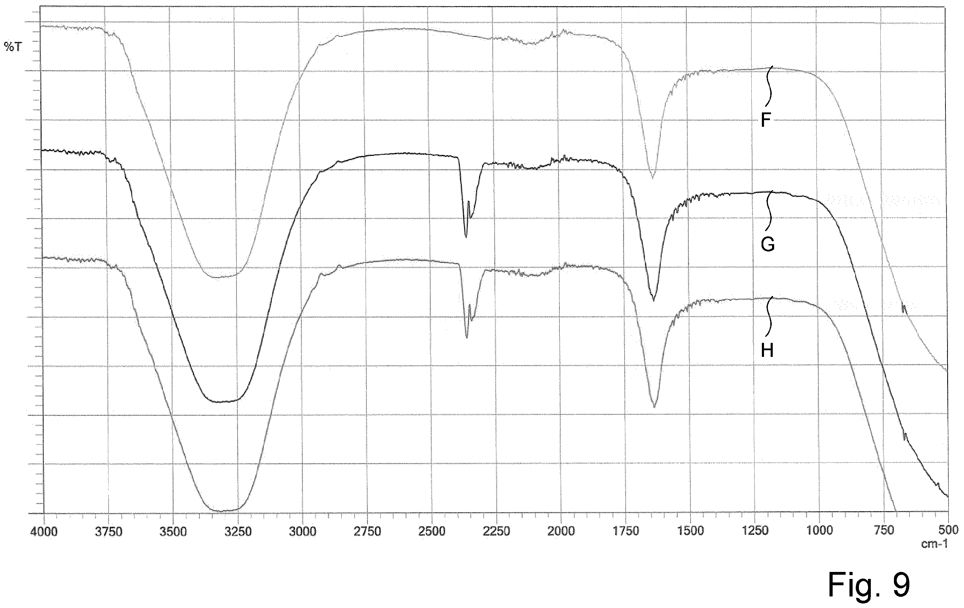

Die Transmissionsspektren sind in Fig. 9 dargestellt, wobei
Spektrum F der Lösung des erfindungsgemäßen Komplexes nach 5.3,
Spektrum G der Lösung des Chitosan-Salicylats mit peripher vorliegendem PHMB nach 5.2 und
Spektrum H der Lösung des Chitosan-Salicylats gemäß 5.1 entspricht.

Bezüglich der dargestellten Transmissionsspektren werden folgende Bandenzuordnungen der Wellenzahlen vorgenommen:
(1) 1300-1750: Hier werden aminofunktionell-bedingte Transmissionen angenommen.
(2) 2250-2500: Hier werden carboxylat-bedingte Transmissionen angenommen.
(3) 2750-3750: Hier werden carboxylat- sowie aminofunktionell-bedingte Transmissionen angenommen.

Bezüglich des Spektrums F, d.h. des erfindungsgemäßem Komplexes, ist festzustellen, dass die Transmissionssektion (2) nicht vorliegt. In der Folge liegt hier allein durch die Umsetzung mit PHMB überraschenderweise eine carboxylat-bedingte Transmissionslücke vor, die als Vorliegen eines Trikomplexes in Form eines labilen Gleichgewichts gedeutet wird.

### 5.4 Untersuchung der Filmbildungseigenschaften

Neben der IR-spektroskopischen Untersuchung der Komplexbildung werden die drei Formulierungen gemäß 5.1 bis 5.3 auch hinsichtlich ihrer Filmbildungseigenschaften untersucht.

Auf einem Mikroskopträger aus Glas wird zunächst zur Abgrenzung jeweils ein Klebestreifen aufgebracht und anschließend mittels einer Pipette tropfenweise Material auf den Glaskörper appliziert, so dass die Fläche vollflächig benetzt ist. Nach einer Wartezeit von 30 Minuten, während welcher die Proben trocknen, werden die Proben mit einem 3D-Konfokal-Mikroskop untersucht.

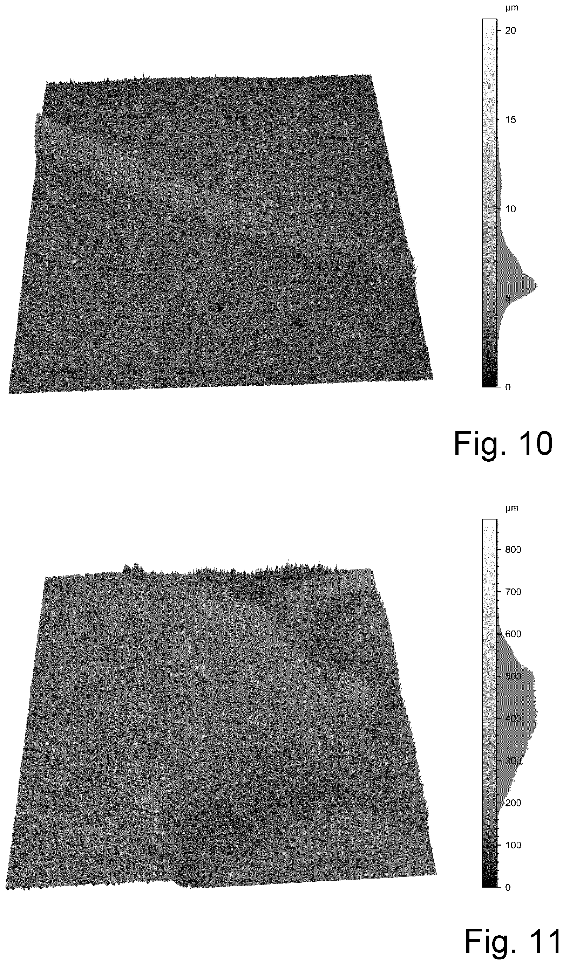

Die Untersuchungsergebnisse sind in den Figuren 10 bis 12 dargestellt.

Es zeigt Fig, 10 ein 3D-Höhenbild einer Probe des Chitosan-Salicylats gemäß 5.1.

Es zeigt Fig. 11 ein 3D-Höhenbild einer Probe des Chitosan-Salicylats mit peripher voliegendem PHMB gemäß 5.2.

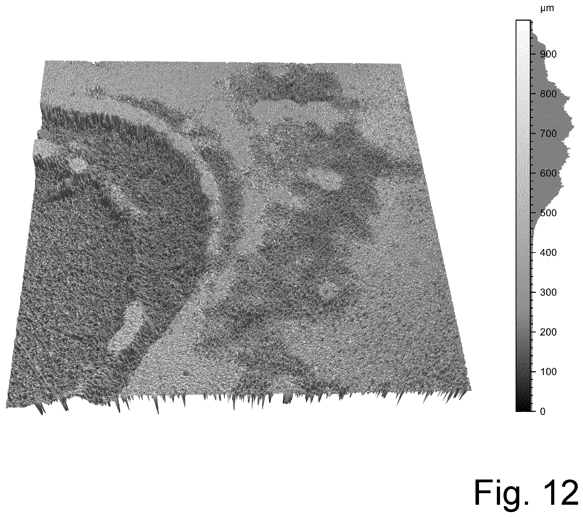

Es zeigt Fig. 12 ein 3D-Höhenbild einer Probe des erfindungsgemäßen Komplexes gemäß 5.2.

Es zeigt sich deutlich, dass nur bei der Probe des erfindungsgemäßen Komplexes eine Filmbildung stattfindet. Nur in diesem Fall bildet sich eine entsprechende Schicht aus, was an den Höhenwerten zu erkennen ist.

Darüber hinaus sind die Proben des erfindungsgemäßen Komplexes im Gegensatz zu den übrigen Proben farblos.

## Patentansprüche

1. Wässrige Zusammensetzung, insbesondere für Hygiene- und/oder Kosmetikanwendungen,
**dadurch gekennzeichnet,**
**dass** die Zusammensetzung einen Komplex aus
a) mindestens einem Polysaccharid, welches Monomereinheiten aus gegebenenfalls substituierten Aminozuckern, insbesondere Glucosaminen und/oder Galactosaminen, aufweist,
b) mindestens einer Carbonsäure und
c) mindestens einem Polyamin und/oder einem Guanid, insbesondere Biguanid, aufweist.

2. Wässrige Zusammensetzung nach Anspruch 1, **dadurch gekennzeichnet, dass** der Komplex in der Zusammensetzung gelöst oder dispergiert vorliegt und/oder
dass die Zusammensetzung, insbesondere die Lösung oder Dispersion, in Form eines Gels, insbesondere eines stabilen Gels, vorliegt.

3. Wässrige Zusammensetzung nach Anspruch 1 oder 2, **dadurch gekennzeichnet, dass** das Polysaccharid ausgewählt ist aus der Gruppe der Glykosaminoglykane und Polyglucosamine sowie deren Mischungen und Derivaten.

4. Wässrige Zusammensetzung nach einem der vorangehenden Ansprüche, **dadurch gekennzeichnet, dass** das Polysaccharid aus gewählt ist aus der Gruppe von Chitosan, Hyaluronsäure, Heparin, Chondroitinsulfat, Keratanasulfat sowie deren Mischungen und Derivaten, insbesondere Chitosan und Hyaluronsäure sowie deren Mischungen und Derivaten, vorzugsweise Chitosan und Chitosanderivaten.

5. Wässrige Zusammensetzung nach einem der vorangehenden Ansprüche, **dadurch gekennzeichnet,**
**dass** die Carbonsäure ausgewählt ist aus Polycarbonsäuren, Hydroxycarbonsäuren und deren Mischungen, insbesondere aromatischen Polycarbonsäuren, aromatischen Hydroxycarbonsäuren oder deren Mischungen und/oder dass das Polyamin ein aliphatisches Polyamin ist und/oder dass das Guanid, insbesondere Biguanid, ein aliphatisches Guanid, insbesondere Biguanid, ist.

6. Wässrige Zusammensetzung nach einem der vorangehenden Ansprüche, **dadurch gekennzeichnet, dass** die Zusammensetzung den Komplex in Mengen von 0,05 bis 10 Gew.-%, insbesondere 0,1 bis 5 Gew.-%, vorzugsweise 0,2 bis 3 Gew.- %, bevorzugt 0,3 bis 2 Gew.-%, besonders bevorzugt 0,4 bis 1 Gew.-%, bezogen auf die Masse der Zusammensetzung, enthält.

7. Wässrige Zusammensetzung nach einem der vorangehenden Ansprüche, **dadurch gekennzeichnet,**
**dass** die Zusammensetzung das Polysaccharid in Mengen von 0,04 bis 9 Gew.-%, insbesondere 0,1 bis 4 Gew.-%, vorzugsweise 0,2 bis 2 Gew.-%, bevorzugt 0,3 bis 1 Gew.-%, besonders bevorzugt 0,4 bis 0,8 Gew.-%, bezogen auf die Masse der Zusammensetzung, enthält und/oder
**dass** die Zusammensetzung die Carbonsäure in Mengen von 0,02 bis 9 Gew.-%, insbesondere 0,02 bis 4 Gew.-%, vorzugsweise 0,1 bis 2 Gew.-%, bevorzugt 0,1 bis 1 Gew.-%, besonders bevorzugt 0,2 bis 0,8 Gew.-%, bezogen auf die Masse der Zusammensetzung, enthält und/oder
**dass** die Zusammensetzung das Polyamid und/oder das Guanid, insbesondere Biguanid, in Mengen von 0,01 bis 1 Gew.-%, insbesondere 0,02 bis 0,5 Gew.-%, vorzugsweise 0,03 bis 0,3 Gew.-%, bevorzugt 0,04 bis 0,2 Gew.-%, besonders bevorzugt 0,05 bis 0,1 Gew.-%, bezogen auf die Masse der Zusammensetzung, enthält.

8. Wässrige Zusammensetzung nach einem der vorangehenden Ansprüche, **dadurch gekennzeichnet, dass** die Zusammensetzung und/oder der Komplex ein gewichtsbezogenes Verhältnis von Polysaccharid zu Carbonsäure im Bereich von 1 : 2 bis 5 : 1, insbesondere 1 : 1,5 bis 3 : 1, vorzugsweise 1 : 1 bis 2,5 : 1, bevorzugt 1,1 : 1 bis 2 : 1, aufweist.

9. Wässrige Zusammensetzung nach einem der vorangehenden Ansprüche, **dadurch gekennzeichnet,**
**dass** die Zusammensetzung einen Alkohol enthält und/oder
**dass** die Zusammensetzung ein Tensid enthält.

10. Verwendung einer wässrigen Zusammensetzung nach einem der Ansprüche 1 bis 9 in oder als Kosmetika, insbesondere insbesondere in Produkten zur Haut- oder Haarpflege.

11. Verwendung einer wässrigen Zusammensetzung nach einem der Ansprüche 1 bis 9 in oder als Desinfektionsmittel, insbesondere zur Desinfektion von Oberflächen.

12. Kosmetische Zubereitung, enthaltend eine wässrige Zusammensetzung nach einem der Ansprüche 1 bis 9.

13. Desinfektionsmittel, enthaltend oder bestehend aus einer wässrigen Zusammensetzung nach einem der Ansprüche 1 bis 9.

14. Verfahren zur Herstellung einer wässrigen Zusammensetzung nach einem der Ansprüche 1 bis 9,
**dadurch gekennzeichnet,**
**dass** in einem ersten Verfahrenschritt (i)
a) ein Polysaccharid, welches Monomereinheiten aus ggf. substituierten Aminozuckern, insbesondere Glucosaminen und oder Galactosaminen, aufweist, und
b) eine Carbonsäure
mit Wasser gemischt und unter Erwärmen gelöst und/oder fein dispergiert werden.

15. Verfahren nach Anspruch 14, **dadurch gekennzeichnet, dass** in einem auf den zweiten Verfahrensschritt (ii) folgenden dritten Verfahrensschritt (iii) die Lösung und/oder Dispersion mit einem einem Polyamin und/oder einem Guanid, insbesondere Biguanid, versetzt wird.
